# EUROPEAN PATENT SPECIFICATION

(11) **EP 1 658 053 B1**
(45) Date of publication and mention of the grant of the patent: **27.02.2008**
(21) Application number: 04786037.4
(22) Date of filing: 23.07.2004
(51) Int. Cl.: A61K 9/14, A61K 9/19, A61K 9/10

(54) **NOVEL COMPOSITIONS OF SILDENAFIL FREE BASE**
NEUE ZUSAMMENSETZUNGEN VON SILDENAFIL-FREIER BASE
NOUVELLES COMPOSITIONS DE BASE LIBRE DE SILDENAFIL

(30) Priority: 23.07.2003 US 489101 P
(43) Date of publication of application: 24.05.2006
(73) Proprietor: Elan Pharma International Limited, Athlone Westmeath (IE)
(72) Inventor: RYDE, Tuula, A., Malvern, PA 19355 (US); HOVEY, Douglas, C., Gilbertsville, Pennsylvania 19525 (US); BOSCH, H., William, Bryn Mawr, PA 19010 (US)
(74) Representative: Wichmann, Hendrik
(86) International application number: PCT/US2004/019106
(87) International publication number: WO 2005/013937

(56) References cited:
- EP-A- 0 960 621
- EP-A- 1 097 711
- WO-A-01/35926
- WO-A-02/05820
- US-A1- 2003 017 120
- US-B1- 6 531 114

## Description

### RELATED APPLICATIONS

The present application claims the benefit of U.S. Provisional Patent Application No. 60/489,101, filed on July 23, 2003.

### FIELD OF THE INVENTION

The present invention relates to compositions of sildenafil free base having an effective average particle size of less than about 2 microns. Preferably, the compositions also comprise at least one surface stabilizer associated with the surface of the sildenafil free base particles. The compositions of the invention surprisingly substantially eliminate fed/fasted variability of absorption of the drug.

### BACKGROUND OF THE INVENTION

### A. Background Regarding Nanoparticulate Compositions

Nanoparticulate compositions, first described in U.S. Patent No. 5,145,684 ("the '684 patent"), are particles consisting of a poorly soluble therapeutic or diagnostic agent having associated with the surface thereof a non-crosslinked surface stabilizer. The '684 patent does not describe nanoparticulate compositions of sildenafil, including sildenafil free base.

Methods of making nanoparticulate compositions are described, for example, in U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" and U.S. Patent No. 5,510,118 for "Process of Preparing Therapeutic Compositions Containing Nanoparticles." These patents do not describe methods of making nanoparticulate sildenafil, including sildenafil free base.

Nanoparticulate compositions are also described, for example, in U.S. Patent Nos. 5,298,262 for "Use of Ionic Cloud Point Modifiers to Prevent Particle Aggregation During Sterilization;" 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray imaging Using Iodinated Aromatic Propanedioates;" 5,336,507 for "Use of Charged Phospholipids to Reduce Nanoparticle Aggregation;" 5,340,564 for "Formulations Comprising Olin 10-G to Prevent Particle Aggregation and Increase Stability;" 5,346,702 for "Use of Non-Ionic Cloud Point Modifiers to Minimize Nanoparticulate Aggregation During Sterilization;" 5,349,957 for "Preparation and Magnetic Properties of Very Small Magnetic-Dextran Particles;" 5,352,459 for "Use of Purified Surface Modifiers to Prevent Particle Aggregation During Sterilization;" 5,399,363 and 5,494,683, both for "Surface Modified Anticancer Nanoparticles;" 5,401,492 for "Water Insoluble NonMagnetic Manganese Particles as Magnetic Resonance Enhancement Agents;" 5,429,824 for "Use of Tyloxapol as a Nanoparticulate Stabilizer;" 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,440 for "Formulations of Oral Gastrointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,683 for "Nanoparticulate Diagnostic Mixed Carbamic Anhydrides as X-Ray Contrast Agents for Blood Pool and Lymphatic System Imaging;" 5,500,204 for "Nanoparticulate Diagnostic Dimers as X-Ray Contrast Agents for Blood Pool and Lymphatic System Imaging;" 5,518,738 for "Nanoparticulate NSAID Formulations;" 5,521,218 for "Nanoparticulate lododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast Compositions Containing Nanoparticles;" 5,552,160 for "Surface Modified NSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabilizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;" 5,573,750 for "Diagnostic Imaging X-Ray Contrast Agents;" 5,573,783 for "Redispersible Nanoparticulate Film Matrices With Protective Overcoats;" 5,580,579 for "Site-specific Adhesion Within the Gl Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydroxypropyl Cellulose as Dispersion Stabilizer;" 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-Ray Contrast Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of Ibuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 "Nanocrystalline Formulations of Human Immunodeficiency Virus (HIV) Protease Inhibitors Using Cellulosic Surface Stabilizers;" 6,068,858 for "Methods of Making Nanocrystalline Formulations of Human Immunodeficiency Virus (HIV) Protease Inhibitors Using Cellulosic Surface Stabilizers;" 6,153,225 for "Injectable Formulations of Nanoparticulate Naproxen;" 6,165,506 for "New Solid Dose Form of Nanoparticulate Naproxen;" 6,221,400 for "Methods of Treating Mammals Using Nanocrystalline Formulations of Human Immunodeficiency Virus (HIV) Protease Inhibitors;" 6,264,922 for "Nebulized Aerosols Containing Nanoparticle Dispersions;" 6,267,989 for "Methods for Preventing Crystal Growth and Particle Aggregation in Nanoparticle Compositions;" 6,270,806 for "Use of PEG-Derivatized Lipids as Surface Stabilizers for Nanoparticulate Compositions;" 6,316,029 for "Rapidly Disintegrating Solid Oral Dosage Form," 6,375,986 for "Solid Dose Nanoparticulate Compositions Comprising a Synergistic Combination of a Polymeric Surface Stabilizer and Dioctyl Sodium Sulfosuccinate," 6,428,814 for "Bioadhesive nanoparticulate compositions having cationic surface stabilizers;" 6,431,478 for "Small Scale Mill;" 6,432,381 for "Methods for Targeting Drug Delivery to the Upper and/or Lower Gastrointestinal Tract;" 6,592,903 for "Nanoparticulate Dispersions Comprising a Synergistic Combination of a Polymeric Surface Stabilizer and Dioctyl Sodium Sulfosuccinate," 6,582,285 for "Apparatus for sanitary wet milling;" 6,656,504 for "Nanoparticulate Compositions Comprising Amorphous Cyclosporine;" 6,742,734 for "System and Method for Milling Materials;" and 6,745,962 for "Small Scale Mill and Method Thereof;" all of which are specifically incorporated by reference. In addition, U.S. Patent Application No. 20020012675 A1, published on January 31, 2002, for "Controlled Release Nanoparticulate Compositions," and WO 02/098565 for "System and Method for Milling Materials," describe nanoparticulate active agent compositions, and are specifically incorporated by reference. None of these references describe nanoparticulate compositions of sildenafil, including sildenafil free base.

Amorphous small particle compositions are described, for example, in U.S. Patent Nos. 4,783,484 for "Particulate Composition and Use Thereof as Antimicrobial Agent;" 4,826,689 for "Method for Making Uniformly Sized Particles from Water-Insoluble Organic Compounds;" 4,997,454 for "Method for Making Uniformly-Sized Particles From Insoluble Compounds;" 5,741,522 for "Ultrasmall, Non-aggregated Porous Particles of Uniform Size for Entrapping Gas Bubbles Within and Methods;" and 5,776,496, for "Ultrasmall Porous Particles for Enhancing Ultrasound Back Scatter." These references do not describe nanoparticulate sildenafil free base.

### B. Background Regarding Sildenafil Free Base and Sildenafil Citrate

Sildenafil, including sildenafil free base and sildenafil citrate, is a phosphodiesterase 5 (PDE5) inhibitor. PDE5 is a cyclic guanosine-3',5'-monophosphate (cGMP)-specific phosphodiesterase belonging to a class of phosphodiesterases which regulate various cell functions by catalyzing the hydrolysis of the second messenger molecules (cGMP) and cyclic adenosine-3',5'-monophosphate (cAMP). Boolell et al., *Int'l J. Impot. Res., 8*:47 (1996).

Sildenafil, also known as 1-[[3-(6,7-dihydro-1-methyl-7-oxo-3-propyl-1*H*-pyrazolo[4,3-*d*]pyrimidin-5-yl)-4-ethoxyphenyl]sulfonyl]-4-methylpiperazine, as well as methods of preparing this compound and salts and derivatives thereof, and methods of use for treatment of erectile dysfunction (ED), are discussed in U.S. Patent Nos. 5,250,534 for "Pyrazolopyrimidinone Antianginal Agents" and 6,469,012 for "Pyrazolopyrimidinones for the Treatment of Impotence." *See also* EP 463,756.

Sildenafil free base has the following structure:

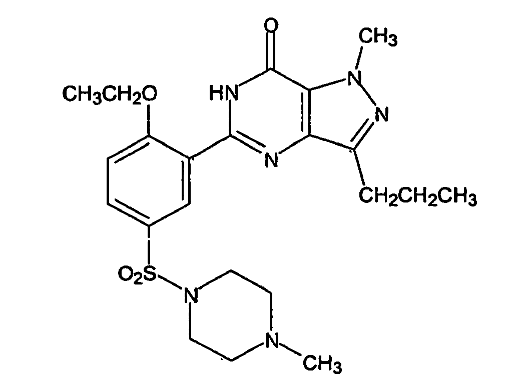

According to the *Physician's Desk Reference* (2003), sildenafil citrate has the following chemical structural formula:

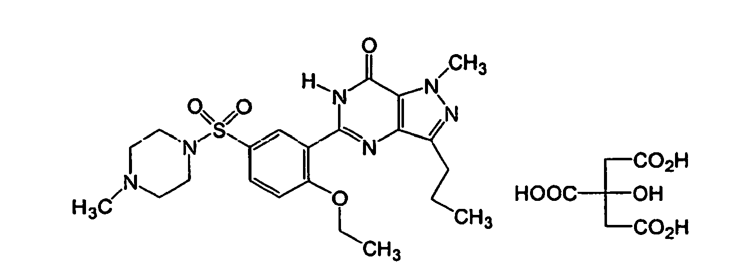

Sildenafil, including sildenafil free base and sildenafil citrate, is a selective inhibitor of cGMP-specific PDE5. See *The Physicians' Desk Reference,* 55^{th} Ed., pp. 2454 (2001). Sildenafil shows the following selectivity with respect to PDE5: > 80-fold for PDE1, >1000-fold for PDE2 and PDE4, and >~4,000-fold for PDE3. Sildenafil is only approximately 10-fold as potent for PDE5 as compared to PDE6, however, and the inhibition of PDE6 has caused visual disturbances in some subjects.

Sildenafil citrate, which is marketed under the tradename VIAGRA® (Pfizer, Inc., USA) in 25, 50, and 100 mg tablets, is also known as 1-[[3-(6,7-dihydro-1-methyl-7-oxo-3-propyl-1*H*-pyrazolo[4,3-*d*]pyrimidin-5-yl)-4-ethoxyphenyl]sulfonyl]-4 methylpiperazine citrate.

Sildenafil citrate has a an empirical formula of C₂₂H₃₀N₆O₄S·C₆H₈O₇ and a molecular weight of 666.71. *See* The Physicians' Desk Reference, 55th Ed., pp. 2534 (2001); and The Merck Index, 13th Ed., pp. 1523 (Merck & Co. 2001). Sildenafil citrate has a solubility of 3.5 mg/mL in water. *See The Physicians' Desk Reference* at pp.2534.

The absolute bioavailability of sildenafil citrate is 40% and the pharmacokinetics are dose-proportional in the range of 25-100 mg. *See* The Physicians' Desk Reference, 55th Ed., pp. 2535 (2001).

In healthy adult males, a 100 mg oral dose of sildenafil citrate results in a mean Cₘₐₓ of 440 ng/mL, a Tₘₐₓ of 60 minutes, and a t_{1/2} of about 4 hours. Absorption takes place mainly from the small intestine, thus gastric emptying is important in the onset of action. Administration of sildenafil citrate with a high fat meal causes a mean delay in Tₘₐₓ of 60 minutes and a mean reduction in Cₘₐₓ of 29%. *See* The Physicians' Desk Reference, 55th Ed., pp. 2535 (2001).

Sildenafil citrate has been tested and approved by the FDA for relief treatment of ED in males only; however, it also may be useful in treating female sexual dysfunction and is currently being tested for this purpose.

The side effects associated with sildenafil citrate include headache, flushing, visual effects, and dyspepsia. Sildenafil citrate is contraindicated in subjects taking organic nitrates.

### 1. Sildenafil and Sexual Dysfunction

PDE5, which is specifically inhibited by sildenafil, is implicated in the mechanisms of both male and female sexual dysfunction. Research related to male sexual dysfunction focuses mainly on ED, which is defined as the persistent inability of a man to achieve and/or maintain an erection adequate for satisfactory sexual performance. NIH Consensus Statement of Impotence, Int'l J. Impot. Res., 5:181 (1993). Sildenafil citrate (VIAGRA®; Pfizer, Inc., USA) is the first, and currently the only, PDE5 inhibitor approved by the FDA for the treatment of ED. Sildenafil citrate is not presently approved for use in treating female sexual dysfunction; however, this use of sildenafil citrate is currently being evaluated in clinical trials.

ED affects an estimated 152 million men worldwide. Aytac et al., BJU Int'l, 84:50 (1999). Between 2 and 7% of the human male population suffers from ED, with the prevalence increasing with age. Leu, N. Engl. J. Med., 342:1802 (2000). In the United States alone, for example, it is estimated that there are up to 30 million males currently afflicted with ED, and 617,000 new cases of ED are expected annually in men between the ages of 40 and 69. *NIH Publication No.* 95-3923 (1995). In addition to age, other factors that increase the likelihood of ED are heart disease, hypertension, diabetes, and medications associated with these conditions. Feldman et al., J. Urol., 151:54 (1994).

Because up to 80% ED cases have a physiological etiology, it is desirable to effectively manage ED by targeting the physiological source of the problem. *NIH Publication No.* 95-3923 (1995). Sildenafil, which is a selective inhibitor of PDE5, is useful in treating ED. Boolell et al., Br. J. Urol., 78:257-61 (1996).

In the healthy male, sexual stimulation triggers release of nitric oxide (NO) from the non-adrenergic, non-cholinergic neurons in the corpus cavernosum of the penis. NO activates guanylate cyclase, which catalyzes the conversion of 5-guanosine triphosphare (5-GTP) to 3', 5'-cGMP. cGMP mediates intracellular signal transduction via a protein activation cascade. This cell signaling cascade results in reduced intracellular Ca²⁺ concentrations causing relaxation of the smooth muscles in the penis, vasodilation in the corpus cavernosum, and ultimately erection. Moreland et al., JPET, 296:225 (2001).

Inhibition of PDE5 by sildenafil enhances the normal action of NO and cGMP. See Boolell et al., Br. J. Urol., 78:257-61 (1996); and Boolell et al., Int. J. Impot. Res., 8:47-52 (1996). PDE5 inhibition by sildenafil in the corpus cavernosum leads to an increase in the intracellular cGMP level, relaxing the smooth musculature of the corpus cavernosum and allowing the flow of blood into the corpus cavernosum resulting in an erection. As such, sildenafil does not cause an erection directly, but rather intensifies the action of the NO released during sexual stimulation. Andersson and Wagner, Physiol Rev., 75:191-236 (1995).

Female sexual dysfunction has only recently been the focus of studies involving sildenafil, but it is now thought to have a physiological basis similar to ED. Female sexual dysfunction is defined as disturbances in sexual desire, arousal, or ability to achieve orgasm. Female sexual dysfunction also includes sexual pain disorders. It is estimated that about 40 million women in the United States experience some form of sexual disorder. In contrast to males, the incidence of female sexual dysfunction does not correlate with age. The incidence of female sexual dysfunction is evenly distributed among women 18 to 59 years of age.

Due to the similarities between the corpus cavernosum and the clitoris with respect to both structure and innervation, it is thought that the mechanism of female sexual dysfunction and, as a result, the treatment of female sexual dysfunction, mirrors that of ED in males.

A sildenafil composition useful in treating sexual dysfunction must have certain features. It must be easily absorbed from the gastrointestinal tract, rapidly reach the target tissue without affecting the vascular system, selectively inhibit PDE5, and have negligible deposition in other tissues. Sildenafil compositions lacking sufficient selectivity for PDE5 as compared to other PDE isoforms can cause unpleasant or potentially dangerous side effects due to inhibition of PDE isoforms responsible for modulating the signal for other cellular processes.

### 2. Sildenafil and Other Conditions

Although the focus of research of sildenafil has been sexual dysfunction, the ability of this compound to effect smooth muscle relaxation makes it useful in treating other conditions. For example, sildenafil can be particularly useful in treating diseases characterized by disorders of gut motility, e.g., irritable bowel syndrome.

### 3. Adverse Properties of Sildenafil Citrate

Despite the demonstrated safety of sildenafil citrate for the treatment of ED, the drug still causes side effects. Subjects report gastrointestinal distress, visual disturbances, flushing, head, back and muscle aches, and hypotension. These side effects are due, in part, to inhibition of PDE isoforms other than PDE5 as well as to the significantly different absorption levels observed between administration under fed and fasted conditions. For example, it is known that the visual disturbances experienced by sildenafil citrate are caused by inhibition of PDE6, and that flushing is due to inhibition of PDE1.

Thus, it is desirable to obtain dosage forms of sildenafil having fewer side effects. In addition, it is desirable to administer the minimum amount of sildenafil required to provide a satisfactory therapeutic response.

Sildenafil is contraindicated for concomitant use of organic nitrates, such as nitroglycerine, because the drug potentiates nitrate-induced vasodilation. The amount of time required to clear sildenafil from a subject's system to allow for safe administration of nitrates is not currently known. Because there is significant overlap between the population most likely to suffer from ED and those who have heart disease and may require use of nitrates, this is of particular concern. Minimizing the dosage of sildenafil will result in faster clearing of the compounds.

### 4. Prior Descriptions Relating to Sildenafil

U.S. Patent No. 6,395,300 for "Porous Drug Matrices and Methods of Manufacture Thereof," describes dosage forms of poorly water soluble drugs, including sildenafil citrate. The dosage form consists of a porous drug matrix of drug microparticles. The drug matrices are made by: (i) dissolving a drug, such as sildenafil citrate, in a volatile solvent to form a drug solution, (ii) combining at least one pore forming agent with the drug solution to form an emulsion, suspension, or second solution, and (iii) removing the volatile solvent and pore forming agent from the emulsion, suspension, or second solution to yield the porous matrix of drug. The pore forming agent can be either a volatile liquid that is immiscible with the drug solvent or a volatile solid compound.

One disadvantage of this dosage form and method of preparation is that the method requires the use of volatile solvents. Solvent precipitation techniques for preparing particles tend to provide particles contaminated with solvents. Such solvents cannot be completely removed by practical manufacturing techniques, and it can be very difficult and expensive, if not impossible, to adequately remove the residual solvents to pharmaceutically acceptable levels.

The amounts of residual solvents present in pharmaceutical products are strictly limited. For example, USP Method 467 specifies that the permissible levels of solvents in pharmaceuticals may not exceed 50 ppm for chloroform and 100 ppm for several other organic volatile impurities. See AN 228-255, "Headspace Analysis of Organic Volatile Impurities in Bulk Pharmaceutical Chemicals," 1-6, at 2 (Hewlett Packard 1995).

Even trace amounts of organic solvents are undesirable because, in addition to lacking any therapeutic benefit, such solvents can be highly toxic. See R. James' "The Toxic Effects of Organic Solvents," Ind. Tox., P.L. Williams et al., Eds., 230-259 (Van Nostrand Reinhold Co., New York, NY 1985). Organic solvents can cause depression of the central nervous systems (CNS) activity and irritation of membranes and tissues. *Id*. at 230-231. Because most organic solvents with few or no functional groups (which usually serve to increase water solubility) are highly lipophobic, they all possess varying degrees of CNS-depressant activity. *Id*. at 231. All organic solvents have some irritant properties. Because cell membranes within the body are largely a protein-lipid matrix, organic solvents are ideal for extracting the fat or lipid portion out of the membrane. This defatting of the skin causes irritation and cell damage, and may seriously injure the skin, lungs, and eyes. After systemic absorption, other acute toxicities are hepatotoxicity (liver toxicity), nephrotoxicity (kidney), and cardiac arrhythmias induced by a sensitization of the heart to catecholamines. *Id.* at 232.

U.S. Patent No. 6,395,300 teaches that nanoparticulate active agents are undesirable, as they "can be difficult to produce and maintain in a stable form due to the tendency of the nanoparticles to flocculate or agglomerate, particularly without the presence of surface modifying agents adsorbed or coated onto the particles." The patent also teaches that milling or wet grinding of pharmaceutical active agents is undesirable "as it can take several days to process a single batch, scaling-up of the milling or grinding process can be difficult and/or costly, the process can be difficult to conduct aseptically, and it is difficult to eliminate shedding of milling media into the product."

U.S. Patent Application Publication No. 20020142050, for "Porous Drug Matrices and Methods of Manufacture Thereof," has the same disclosure as and claims priority of U.S. Patent No. 6,395,300.

U.S. Patent Nos. 6,395,736 and 6,391,869, both for "Compositions and Methods for the Treatment of Anorectal Disorders", refer to compositions and methods for treating anorectal disorders. The compositions comprise a nitric oxide donor in combination with a second agent, which can be a PDE5 inhibitor such as sildenafil.

U.S. Patent No. 6,395,736 teaches that "to increase bioavailability of drugs, to extend therapeutic efficacy, and to improve patient compliance, various dosage forms have been developed over the years. These include .. liposomal and drug delivery via nanoparticles (emulsion, suspension, etc.), and ointment (See Edman, Biopharmaceutics of Ocular Drug Delivery, CRC Press, 1993)." This reference to "nanoparticles" does not teach the nanoparticulate compositions of the present invention; rather, it refers to lipid nanoparticles containing solubilized drug.

U.S. Patent Application Publication No. 20020003179, for "Media Milling," describes a process for preparing a dispersion of solid particles of a milled substrate in a fluid carrier. An example of a compound to be milled is sildenafil. The method comprises utilizing two sizes of milling media: large and small (the use of multiple sizes of grinding media to mill active agents is also taught, for example, in U.S. Patent No. 6,431,478 for "Small Scale Mill."). The reference does not teach milling of sildenafil free base, nor does it teach a sildenfil composition having a minimal difference in pharmacokinetic profiles when administered under fed as compared to fasted conditions.

There is a need in the art for sildenafil compositions which overcome these and other problems associated with prior sildenafil formulations. The present invention satisfies these needs.

### SUMMARY OF THE INVENTION

The present invention relates to compositions comprising sildenafil free base particles having an effective average particle size of less than about 2 microns. Preferably, the compositions comprise particles of sildenafil free base and at least one surface stabilizer associated with the surface of the sildenafil free base particles. The nanoparticulate sildenafil free base compositions substantially eliminate the effect of food on the pharmacokinetic profiles of the compositions.

The present invention also relates to compositions of nanoparticulate sildenafil free base in combination with one or more non-nanoparticulate (*i.e.*, solubilized or microparticulate) sildenafil or non-sildenafil compounds, or nanoparticulate non-sildenafil free base active agents. If such other non-sildenafil free base active agents have a nanoparticulate particle size, then preferably such other non-sildenafil free base active agents have one or more surface stabilizers associated with the surface of the active agent.

Another aspect of the invention is directed to pharmaceutical compositions comprising a sildenafil free base composition of the invention. The pharmaceutical compositions preferably comprise particles of sildenafil free base having an effective average particle size of less than about 2 microns and a pharmaceutical acceptable carrier, as well as any desired excipients. Preferably, the compositions also comprise at least one surface stabilizer associated with the surface of the sildenafil free base particles. Such pharmaceutical compositions can additionally comprise one or more of the following: (1) non-nanoparticulate sildenafil (free base and citrate forms), (2) non-nanoparticulate non-sildenafil free base active agents, or (3) nanoparticulate non-sildenafil free base active agents.

This invention is also directed to a method of making a nanoparticulate sildenafil free base composition according to the invention. Such a method comprises contacting sildenafil free base and at least one surface stabilizer for a time and under conditions sufficient to provide a sildenafil free base composition having an effective average particle size of less than about 2 microns. The one or more surface stabilizers can be contacted with sildenafil free base either before, preferably during, or after particle size reduction. In addition, one or more non-sildenafil free base active agents can be reduced in size at the same time as sildenafil free base, to produce a nanoparticulate sildenafil free base composition and nanoparticulate non-sildenafil free base active agent composition. A non-sildenafil free base active agent, which is either non-nanoparticulate (solubilized or microparticulate) or nanoparticulate, can also be added to the nanoparticulate sildenafil free base composition after particle size reduction.

The present invention is directed to methods of using the nanoparticulate sildenafil free base compositions of the invention for conditions in which a PDE5 inhibitor is prescribed. Such conditions include, but are not limited to, for example, male erectile dysfunction, impotence, female sexual dysfunction, clitoral dysfunction, female hypoactive sexual desire disorder, female sexual arousal disorder, female sexual pain disorder, female sexual orgasmic dysfunction, and sexual dysfunction due to spinal cord injury.

Other conditions that may be treated using the nanoparticulate sildenafil free base compositions of the invention include, but are not limited to, premature labor, dysmenorrhea, benign prostatic hyperplasia, bladder outlet obstruction, incontinence, stable, unstable and variant (Prinzmetal) angina, hypertension, pulmonary hypertension, chronic obstructive pulmonary disease, coronary artery disease, congestive heart failure, atherosclerosis, conditions of reduced blood vessel patency, post-percutaneous transluminal coronary angioplasty, peripheral vascular disease, stroke, nitrate induced tolerance, bronchitis, allergic asthma, chronic asthma, allergic rhinitis, glaucoma, diseases characterized by disorders of gut motility, irritable bowel syndrome, pre-eclampsia, Kawasaki's syndrome, nitrate tolerance, multiple sclerosis, diabetic nephropathy, peripheral diabetic neuropathy, Alzheimer's disease, acute respiratory failure, psoriasis, skin necrosis, cancer, metastasis, baldness, nutcracker oesophagus, anal fissure, hemorrhoids, and hypoxic vasoconstriction.

Such methods comprise administering to a subject a therapeutically effective amount of a nanoparticulate sildenafil free base composition according to the invention. Alternatively, such methods comprise administering to a subject a therapeutically effective amount of a nanoparticulate sildenafil free base composition according to the invention in combination with one or more non-sildenafil free base agents. Such non-sildenafil free base active agents can be either non-nanoparticulate (solubilized or microparticulate) or nanoparticulate.

Both the foregoing general description and the following brief description of the drawings and detailed description are exemplary and explanatory and are intended to provide further explanation of the invention as claimed. Other objects, advantages, and novel features will be readily apparent to those skilled in the art from the following detailed description of the invention.

### BRIEF DESCRIPTION OF THE FIGURES

Figure 1: shows the mean sildenafil concentration (ng/mL) for Formulation #2 (nanoparticulate sildenafil free base), Formulation #4 (nanoparticulate sildenafil citrate), and Formulation #5 (microparticulate sildenafil citrate; Viagra®), given under fed and fasted conditions, over a 2 hour time period; and

Figure 2: shows the mean sildenafil concentration (ng/mL) for Formulation #2 (nanoparticulate sildenafil free base), Formulation #4 (nanoparticulate sildenafil citrate), and Formulation #5 (microparticulate sildenafil citrate; Viagra®), given under fed and fasted conditions, over an 8 hour time period.

### DETAILED DESCRIPTION OF THE INVENTION

The present invention is directed to compositions comprising particles of sildenafil free base having an effective average particle size of less than about 2 microns. Preferably, the sildenafil free base particles have one or more surface stabilizers associated with the surface of the sildenafil free base particles.

It was surprisingly discovered that the nanoparticulate sildenafil free base compositions of the invention demonstrate substantially similar pharmacokinetic profiles when administered under fed and fasted conditions.

In another embodiment of the invention, preferably the compositions of the invention are bioequivalent when administered under fed and fasted conditions. Under U.S. Food and Drug Administration (USFDA) guidelines, two products or methods are bioequivalent if the 90% Confidence Intervals (CI) for Cₘₐₓ (peak concentration) and the AUC (area under the concentration/time curve) are between 0.80 to 1.25. For Europe, the test for bioequivalency is if two products or methods have a 90% Cl for AUC of between 0.80 to 1.25 and a 90% Cl for Cₘₐₓ of between 0.70 to 1.43. (Tₘₐₓ is not relevant to bioequivalency determinations under USFDA and European regulatory guidelines.)

This is significant, as it means that there is no substantial difference in the quantity of sildenafil free base absorbed, or the rate of sildenafil free base absorption, when the nanoparticulate sildenafil free base compositions are administered in the fed versus the fasted state. Therefore, the nanoparticulate sildenafil free base compositions of the invention can substantially eliminate the effect of food on the pharmacokinetics of the drug.

Prior to the present invention, it was known that non-nanoparticulate forms of sildenafil citrate (*i.e.,* Viagra®) exhibit a substantial difference in drug absorption when administered under fed as compared to fasted conditions. It was surprisingly discovered that this same fed/fasted variability is observed with nanoparticulate forms of sildenafil citrate; *only* nanoparticulate sildenafil free base dosage forms exhibited a substantially similar pharmacokinetic profile when administered under fed and fasted conditions.

As described in the examples below, Viagra® administered under fed and fasted conditions exhibited an AUC_{inf} of 2514 ng/mL·h and 1749.6 ng/mL·h, respectively - a difference of 764.4 ng/mL·h or ~ 30%. A nanoparticulate sildenafil citrate composition administered under fed and fasted conditions exhibited an AUC_{inf} of 1335.2 ng/mL·h and 897.8 ng/mL·h, respectively - a difference of 437.4 ng/mL·h or - 33%. In surprising contrast to these results, a nanoparticulate sildenafil free base composition, administered under fed and fasted conditions, exhibited an AUC_{inf} of 2127.8 ng/mL·h and 2105.3 ng/mL·h, respectively - a difference of 22.5 ng/mL·h or about 1%. A difference of 22.5 ng/mL·h is almost *40 times* less than the fed/fasted variability observed with Viagra®, and over *19 times* less than the fed/fasted variability observed with a nanoparticulate sildenafil citrate composition.

Moreover, as further described in the examples below, the Tₘₐₓ and Cₘₐₓ profiles for the nanoparticulate sildenafil free base composition were substantially similar when administered under fed and fasted conditions: ~ 11 % difference for Cₘₐₓ and a 4% difference for Tₘₐₓ. In contrast, both the Viagra® and nanoparticulate sildenafil citrate compositions showed dramatically different results for Tₘₐₓ and Cₘₐₓ when the formulations were administered under fed as compared to fasted conditions: for Viagra®, a ~23% difference in Cₘₐₓ and 60% difference in Tₘₐₓ, and for the nanoparticulate sildenafil citrate composition, a 49% difference in Cₘₐₓ and 52% difference in Tₘₐₓ.

Benefits of a dosage form which substantially eliminates the effect of food include a safer dosage form, as well as an increase in subject convenience, thereby increasing subject compliance, as the subject does not need to ensure that they are taking a dose either with or without food.

Preferably, the difference in the AUC of the nanoparticulate sildenafil free base compositions of the invention, when administered in the fed versus the fasted state, is less than about 35%, less than about 30%, less than about 25%, less than about 20%, less than about 15%, less than about 10%, less than about 5%, less than about 3%, less than about 1%, or essentially no difference.

In addition, preferably the difference in the Cₘₐₓ of the nanoparticulate sildenafil free base compositions of the invention, when administered in the fed versus the fasted state, is less than about 50%, less than about 40%, less than about 30%, less than about 20%, less than about 15%, less than about 10%, less than about 5%, less than about 3%, or essentially no difference.

Finally, preferably the difference in the Tₘₐₓ of the nanoparticulate sildenafil free base compositions of the invention, when administered in the fed versus the fasted state, is less than about 60%, less than about 50%, less than about 40%, less than about 30%, less than about 20%, less than about 15%, less than about 10%, less than about 5%, less than about 3%, or essentially no difference.

Other advantages of the sildenafil free base compositions of the invention as compared to non-nanoparticulate sildenafil compositions and/or nanoparticulate sildenafil citrate compositions may include, but are not limited to: (1) faster onset of action; (2) smaller tablet or other solid dosage form size, or smaller volume if in a liquid dosage form; (3) smaller doses of drug required to obtain the same pharmacological effect; (4) faster clearance from the bloodstream due to the smaller doses, thereby enabling safe administration of nitrates; (5) increased bioavailability; (6) improved pharmacokinetic profiles, such as improved Cₘₐₓ, Tₘₐₓ, and/or AUC profiles; (7) an increased rate of dissolution; (8) bioadhesive sildenafil free base compositions; (9) the compositions do not require the use of potentially toxic solvents; and (10) the sildenafil free base compositions of the invention can be used in conjunction with other active agents.

In addition, the invention encompasses the sildenafil free base compositions of the invention formulated or coadministered with one or more non-sildenafil free base active agents, either non-nanoparticulate (solubilized or microparticulate) or nanoparticulate. Methods of using such combination compositions are also encompassed by the invention.

Nanoparticulate formulations of sildenafil free base can be administered with supplements known to be involved in the biosynthesis of cGMP. For example, L-arginine is involved in the biosynthesis of NO and thus administration of sildenafil free base in combination with L-arginine can enhance the overall efficacy of the composition.

Other exemplary types of active agents which can be used in combination with the nanoparticulate sildenafil free base compositions of the invention are described below. If the non-sildenafil free base active agent has a nanoparticulate particle size, then preferably such a non-sildenafil free base active agent has one or more surface stabilizers associated with the surface of the active agent. The surface stabilizer(s) can be the same as or different from the surface stabilizer(s) associated with the surface of the nanoparticulate sildenafil free base.

The present invention is described herein using several definitions, as set forth below and throughout the application.

"About" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term.

As used herein with reference to stable drug particles, "stable" includes, but is not limited to, one or more of the following parameters: (1) that the sildenafil free base particles do not appreciably flocculate or agglomerate due to interparticle attractive forces, or otherwise significantly increase in particle size over time; (2) that the physical structure of the sildenafil free base particles is not altered over time, such as by conversion from an amorphous phase to a crystalline phase; (3) that the sildenafil free base particles are chemically stable; and/or (4) where sildenafil free base has not been subject to a heating step at or above the melting point of sildenafil free base in the preparation of the nanoparticles of the invention.

"Non-nanoparticulate active agents" refers to compositions of solubilized active agents, or particulate active agents having an effective average particle size of greater than about 2 microns. Nanoparticulate active agents as defined herein have an effective average particle size of less than about 2 microns.

### A. Additional Preferred Characteristics of the Sildenafil Free Base Compositions of the Invention

### 1. Fast Onset of Activity

The use of conventional formulations of sildenafil for the treatment of male and female sexual dysfunction is not ideal due to delayed onset of action. Conventional formulations of sildenafil have a Tₘₐₓ of approximately 60 minutes. Thus, conventional formulations of sildenafil require advance planning of sexual activity, precluding spontaneity. In addition, slow acting conventional formulations of sildenafil encourage unnecessary dosing, due to anticipation of sexual activity that may or may not transpire.

In contrast, the nanoparticulate sildenafil free base compositions of the invention, when formulated into a suitable dosage form enabling fast onset of activity, such as a nasal or pulmonary aerosol spray (either dry powder, non-aqueous, or aqueous), can provide faster therapeutic effects. Such dosage forms of the nanoparticulate sildenafil free base compositions of the invention are useful in treating sexual dysfunction where spontaneity is important and where the potential for pharmacological habituation exists.

### 2. Increased Bioavailability

The sildenafil free base compositions of the invention preferably exhibit increased bioavailability, at the same dose, and require smaller doses as compared to prior non-nanoparticulate sildenafil compositions as well as compared to nanoparticulate sildenafil citrate compositions.

Any drug, including sildenafil, can have adverse side effects. Thus, lower doses of sildenafil which can achieve the same or better therapeutic effects as those observed with larger doses of non-nanoparticulate sildenafil compositions and/or nanoparticulate sildenafil citrate compositions are desired. Such lower doses can be realized with the sildenafil free base compositions of the invention because the greater bioavailability observed with the nanoparticulate sildenafil free base compositions means that smaller does of sildenafil free base are required to obtain the desired therapeutic effect.

### 3. Redispersibility Profiles of the Sildenafil Free Base Compositions of the Invention

An additional feature of the sildenafil free base compositions of the invention is that the compositions redisperse such that the effective average particle size of the redispersed sildenafil free base particles is less than about 2 microns. This is significant, as if upon administration the nanoparticulate sildenafil free base compositions of the invention did not redisperse to a substantially nanoparticulate particle size, then the dosage form may lose the benefits afforded by formulating sildenafil free base into a nanoparticulate particle size.

Nanoparticulate sildenafil free base compositions benefit from the small particle size of sildenafil free base; if the nanoparticulate sildenafil free base particles do not redisperse into the small particle sizes upon administration, then "clumps" or agglomerated sildenafil free base particles are formed, owing to the extremely high surface free energy of the nanoparticulate system and the thermodynamic driving force to achieve an overall reduction in free energy. With the formation of such agglomerated particles, the bioavailability of the dosage form may fall well below that observed with the liquid dispersion form of the nanoparticulate sildenafil free base composition.

Moreover, the nanoparticulate sildenafil free base compositions of the invention preferably exhibit dramatic redispersion of the nanoparticulate sildenafil free base particles upon administration to a mammal, such as a human or animal, as demonstrated by reconstitution/redispersion in a biorelevant aqueous media such that the effective average particle size of the redispersed sildenafil free base particles is less than about 2 microns. Such biorelevant aqueous media can be any aqueous media that exhibit the desired ionic strength and pH, which form the basis for the biorelevance of the media. The desired pH and ionic strength are those that are representative of physiological conditions found in the human body. Such biorelevant aqueous media can be, for example, aqueous electrolyte solutions or aqueous solutions of any salt, acid, or base, or a combination thereof, which exhibit the desired pH and ionic strength.

Biorelevant pH is well known in the art. For example, in the human stomach, the pH ranges from slightly less than 2 (but typically greater than 1) up to 4 or 5. In the human small intestine the pH can range from 4 to 6, and in the human colon it can range from 6 to 8. Biorelevant ionic strength is also well known in the art. Fasted state gastric fluid has an ionic strength of about 0.1M while fasted state intestinal fluid has an ionic strength of about 0.14. *See e.g.,* Lindahl et al., "Characterization of Fluids from the Stomach and Proximal Jejunum in Men and Women," Pharm. Res., 14 (4): 497-502 (1997).

It is believed that the pH and ionic strength of the test solution is more critical than the specific chemical content. Accordingly, appropriate pH and ionic strength values can be obtained through numerous combinations of strong acids, strong bases, salts, single or multiple conjugate acid-base pairs (*i.e*., weak acids and corresponding salts of that acid), monoprotic and polyprotic electrolytes, *etc*.

Representative electrolyte solutions can be, but are not limited to, HCl solutions, ranging in concentration from about 0.001 to about 0.1 M, and NaCl solutions, ranging in concentration from about 0.001 to about 0.1 M, and mixtures thereof. For example, electrolyte solutions can be, but are not limited to, about 0.1 M HCl or less, about 0.01 M HCl or less, about 0.001 M HCl or less, about 0.1 M NaCl or less, about 0.01 M NaCl or less, about 0.001 M NaCl or less, and mixtures thereof. Of these electrolyte solutions, 0.01 M HCl and/or 0.1 M NaCl, are most representative of fasted human physiological conditions, owing to the pH and ionic strength conditions of the proximal gastrointestinal tract.

Electrolyte concentrations of 0.001 M HCl, 0.01 M HCl, and 0.1 M HCl correspond to pH 3, pH 2, and pH 1, respectively. Thus, a 0.01 M HCl solution simulates typical acidic conditions found in the stomach. A solution of 0.1 M NaCl provides a reasonable approximation of the ionic strength conditions found throughout the body, including the gastrointestinal fluids, although concentrations higher than 0.1 M may be employed to simulate fed conditions within the human GI tract.

Exemplary solutions of salts, acids, bases or combinations thereof, which exhibit the desired pH and ionic strength, include but are not limited to phosphoric acid/phosphate salts + sodium, potassium and calcium salts of chloride, acetic acid/acetate salts + sodium, potassium and calcium salts of chloride, carbonic acid/bicarbonate salts + sodium, potassium and calcium salts of chloride, and citric acid/citrate salts + sodium, potassium and calcium salts of chloride.

In other embodiments of the invention, the redispersed sildenafil free base particles of the invention (redispersed in an aqueous, biorelevant, or any other suitable media) have an effective average particle size of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

By "an effective average particle size of less than about 2000 nm" it is meant that at least 50% of the sildenafil free base particles have a particle size less than the effective average, by weight, *i.e.,* less than about 2000 nm, 1900 nm, 1800 nm, *etc.,* when measured by the above-noted techniques. Preferably, at least about 70%, at least about 90%, at least about 95%, or at least about 99% of the sildenafil free base particles have a particle size less than the effective average, *i.e.,* less than about 2000 nm, 1900 nm, 1800 nm, 1700 nm, etc.

Redispersibility can be tested using any suitable means known in the art. See *e.g.,* the example sections of U.S. Patent No. 6,375,986 for "Solid Dose Nanoparticulate Compositions Comprising a Synergistic Combination of a Polymeric Surface Stabilizer and Dioctyl Sodium Sulfosuccinate."

### 4. Bioadhesive Sildenafil Free Base Compositions

Bioadhesive sildenafil free base compositions of the invention comprise at least one cationic surface stabilizer, which are described in more detail below. Bioadhesive formulations of sildenafil free base exhibit exceptional bioadhesion to biological surfaces, such as mucous.

The term bioadhesion refers to any attractive interaction between two biological surfaces or between a biological and a synthetic surface. In the case of bioadhesive nanoparticulate sildenafil free base compositions, the term bioadhesion is used to describe the adhesion between the nanoparticulate sildenafil free base composition and a biological substrate (*i.e*. gastrointestinal mucin, lung tissue, nasal mucosa, etc.). *See e.g*., U.S. Patent No. 6,428,814 for "Bioadhesive Nanoparticulate Compositions Having Cationic Surface Stabilizers," which is specifically incorporated by reference.

The bioadhesive sildenafil free base compositions of the invention are useful in any situation in which it is desirable to apply the compositions to a biological surface. The bioadhesive sildenafil free base compositions coat the targeted surface in a continuous and uniform film which is invisible to the naked human eye.

A bioadhesive sildenafil free base composition slows the transit of the composition, and some sildenafil free base particles would also most likely adhere to tissue other than the mucous cells and therefore give a prolonged exposure to sildenafil free base, thereby increasing absorption and the bioavailability of the administered dosage.

### 5. Pharmacokinetic Profiles of the Sildenafil Free Base Compositions of the Invention

The present invention provides nanoparticulate sildenafil free base compositions having a desirable pharmacokinetic profile when administered to mammalian subjects. The desirable pharmacokinetic profile can include one or more of the following characteristics: (1) the Tₘₐₓ of an administered dose of a nanoparticulate sildenafil free base composition can be less than that of a non-nanoparticulate sildenafil composition, or less than that of a nanoparticulate sildenafil citrate composition, administered at the same dosage; (2) the Cₘₐₓ of a nanoparticulate sildenafil free base composition can be greater than the Cₘₐₓ of a non-nanoparticulate sildenafil composition, or greater than the Cₘₐₓ of a nanoparticulate sildenafil citrate composition, administered at the same dosage; and/or (3) the AUC of a nanoparticulate sildenafil composition can be greater than the AUC of a non-nanoparticulate sildenafil composition, or greater than the AUC of a nanoparticulate sildenafil citrate composition, administered at the same dosage.

In healthy adult males, a 100 mg oral dose of sildenafil citrate results in a mean Cₘₐₓ of 440 ng/mL and a Tₘₐₓ of 60 minutes.

For nanoparticulate compositions of sildenafil free base according to the invention, a 100 mg oral dose in healthy adult males results in a mean Cₘₐₓ of greater than about 440 ng/mL, a Tₘₐₓ of less than about 60 minutes, or a combination thereof.

In comparative pharmacokinetic testing with a non-nanoparticulate sildenafil composition (*e.g*., Viagra®), or a nanoparticulate composition of sildenafil citrate, a nanoparticulate sildenafil free base composition, administered at the same dosage, preferably exhibits a Tₘₐₓ which is less than about 200%, less than about 175%, less than about 150%, less than about 125%, less than about 100%, less than about 90%, less than about 80%, less than about 70%, less than about 60%, less than about 50%, less than about 40%, less than about 30%, less than about 25%, less than about 20%, less than about 15%, or less than about 10% of the Tₘₐₓ exhibited by the non-nanoparticulate sildenafil composition or nanoparticulate sildenafil citrate composition.

The sildenafil free base compositions of the invention preferably have a Tₘₐₓ of less than about 1.5 hours, less than about 1.25 hours, less than about 1.0 hours, less than about 50 minutes, less than about 40 minutes, less than about 45 minutes, less than about 35 minutes less than about 30 minutes, less than about 25 minutes, less than about 20 minutes, less than about 15 minutes, or less than about 10 minutes.

In comparative pharmacokinetic testing with a non-nanoparticulate sildenafil composition (*e.g*., Viagra®) or a nanoparticulate sildenafil citrate composition, a nanoparticulate sildenafil free base composition, administered at the same dosage, preferably exhibits a Cₘₐₓ which is greater than about 5%, greater than about 10%, greater than about 15%, greater than about 20%, greater than about 30%, greater than about 40%, greater than about 50%, greater than about 60%, greater than about 70%, greater than about 80%, greater than about 90%, greater than about 100%, greater than about 110%, greater than about 120%, greater than about 130%, greater than about 140%, or greater than about 150% than the Cₘₐₓ exhibited by the non-nanoparticulate sildenafil composition or nanoparticulate sildenafil citrate composition.

Following administration of a 100 mg oral dose of a nanoparticulate sildenafil free base composition of the invention, the resulting Cₘₐₓ is preferably greater than about 440 ng/mL, greater than about 450 ng/mL. greater than about 500 ng/mL, greater than about 550 ng/mL, greater than about 600 ng/mL, greater than about 650 ng/mL, greater than about 700 ng/mL, greater than about 750 ng/mL, greater than about 800 ng/mL, greater than about 850 ng/mL, greater than about 900 ng/mL, greater than about 950 ng/mL, greater than about 1000 ng/mL, greater than about 1050 ng/mL, greater than about 1100 ng/mL, greater than about 1150 ng/mL, greater than about 1200 ng/mL, greater than about 1250 ng/mL, greater than about 1300 ng/mL, about 1350 ng/mL, or greater than about 1400 ng/mL.

In comparative pharmacokinetic testing with a non-nanoparticulate sildenafil composition (*e.g*., Viagra®) or a nanoparticulate sildenafil citrate composition, a nanoparticulate sildenafil free base composition, administered at the same dosage, preferably exhibits an AUC which is greater than about 5%, greater than about 10%, greater than about 15%, greater than about 20%, greater than about 30%, greater than about 40%, greater than about 50%, greater than about 60%, greater than about 70%, greater than about 80%, greater than about 90%, greater than about 100%, greater than about 110%, greater than about 120%, greater than about 130%, greater than about 140%, or greater than about 150% than the AUC exhibited by the non-nanoparticulate sildenafil composition or nanoparticulate sildenafil citrate composition.

The desirable pharmacokinetic profile, as used herein, is the pharmacokinetic profile measured after an initial dose of sildenafil free base. The compositions can be formulated in any way as described below.

Any sildenafil free base composition according to the invention, and providing a desired pharmacokinetic profile, is suitable for administration according to the methods of the invention.

### 6. Combination Pharmacokinetic Profile Compositions

In yet another embodiment of the invention, a first sildenafil free base composition providing a desired pharmacokinetic profile is co-administered, sequentially administered, or combined with at least one other sildenafil composition that generates a desired different pharmacokinetic profile. More than two sildenafil compositions can be co-administered, sequentially administered, or combined. While at least one of the sildenafil compositions is a sildenafil free base composition having a nanoparticulate particle size, the additional one or more sildenafil compositions (sildenafil free base, sildenafil citrate, or other suitable sildenafil form) can be nanoparticulate, solubilized, or have a conventional microparticulate particle size.

For example, a first sildenafil free base composition can have a nanoparticulate particle size, conferring a short Tₘₐₓ and typically a higher Cₘₐₓ. This first sildenafil free base composition can be combined, co-administered, or sequentially administered with a second composition comprising: (1) sildenafil free base having a larger (but still nanoparticulate) particle size, and therefore exhibiting slower absorption, a longer Tₘₐₓ, and typically a lower Cₘₐₓ; or (2) a microparticulate sildenafil composition exhibiting a longer Tₘₐₓ, and typically a lower Cₘₐₓ.

The second, third, fourth, *etc*., sildenafil compositions can differ from the first, and from each other, for example, (1) in the effective average particle sizes of each sildenafil composition; (2) in the dosage of sildenafil; and/or (3) in the form of sildonafil utilized. Such a combination composition can reduce the dose frequency required.

Preferably where co-administration of a "fast-acting" formulation and a "longer-lasting" formulation is desired, the two formulations are combined within a single composition, for example a dual-release composition.

### B. Compositions

The invention provides compositions comprising nanoparticulate sildenafil free base particles and, preferably, at least one surface stabilizer. Preferably, the one or more surface stabilizers are associated with the surface of the sildenafil free base particles. Surface stabilizers useful herein preferably do not chemically react with the sildenafil free base particles or itself, but are believed to be adsorbed or associated with the surface of the sildenafil free base particles. Moreover, preferably individual molecules of the surface stabilizer are essentially free of intermolecular cross-linkages.

The present invention includes nanoparticulate sildenafil free base compositions together with one or more non-toxic physiologically acceptable carriers, adjuvants, or vehicles, collectively referred to as carriers. The compositions can be formulated for parenteral injection (*e.g*., intravenous, intramuscular, or subcutaneous), oral (in solid, liquid, or aerosol form), vaginal, nasal, rectal, ocular, local (powders, ointments or drops), buccal, intracisternal, intraperitoneal, or topical administration, and the like.

Nanoparticulate sildenafil free base compositions intended for fast onset of activity are preferably formulated into an aerosol or nasal spray.

### 1. Sildenafil Free Base Particles

"Sildenafil" as used herein encompasses sildenafil free base or a derivative thereof, which is a deprotonated form of a sildenafil salt, and which is a selective inhibitor of cGMP-specific PDE5.

Sildenafil free base has the following structure:

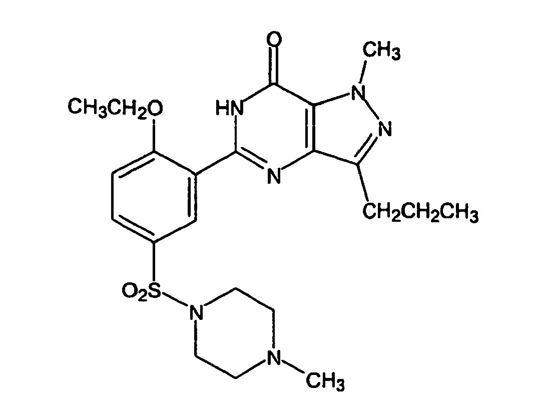

The sildenafil free base particles can be present in a crystalline phase, an amorphous phase, a semi-crystalline phase, a semi-amorphous phase, or a mixture thereof.

Nanoparticulate sildenafil free base compositions are contemplated to be useful in treating a wide range of conditions and disorders for which a potent and selective PDE5 inhibitor is indicated, including but not limited to the prevention or treatment of mammalian sexual disorders and conditions characterized by decreased gut motility. In particular, such compositions have the potential for reduced side effects such as headache, back ache, muscle ache, painful or prolonged erection, priapism, hematuria, dizziness, rash, flushing, dyspepsia, nasal congestion, visual disturbances, and hypotension, when compared to compositions of conventional sildenafil. Thus, nanoparticulate sildenafil free base compositions of the invention are particularly useful as an alternative to conventional sildenafil in subjects who are prone to experience significant side effects from traditional non-nanoparticulate sildenafil compositions.

Furthermore, nanoparticulate sildenafil free base compositions also have the potential for reduced adverse reactions with nitrates because nanoparticulate formulations enable administration of smaller doses of sildenafil free base, thus enabling faster clearance of sildenafil free base from the bloodstream.

### 2. Non-Sildenafil Active Agents

The nanoparticulate sildenafil free base compositions of the invention can additionally comprise another sildenafil form - such as sildenafil citrate - or one or more non-sildenafil active agents, in either a conventional or nanoparticulate particle size. The additional active agents can be present in a crystalline phase, an amorphous phase, a semi-crystalline phase, a semi-amorphous phase, or a mixture thereof.

If the additional active agent has a nanoparticulate particle size, then preferably it will have one or more surface stabilizers associated with the surface of the active agent. In addition, if the active agent has a nanoparticulate particle size, then it is preferably poorly soluble and dispersible in at least one liquid dispersion media. By "poorly soluble" it is meant that the active agent has a solubility in a liquid dispersion media of less than about 30 mg/mL, less than about 20 mg/mL, less than about 10 mg/mL, or less than about 1 mg/mL. Useful liquid dispersion medias include, but are not limited to, water, aqueous salt solutions, safflower oil, and solvents such as ethanol, t-butanol, hexane, and glycol.

Such active agents can be, for example, a therapeutic agent. A therapeutic agent can be a pharmaceutical agent, including biologics such as amino acids, proteins, peptides, and nucleotides. The active agent can be selected from a variety of Known classes of drugs, including, for example, non-sildenafil PDE5 inhibitors, amino acids, proteins, peptides, nucleotides, anti-obesity drugs, central nervous system stimulants, carotenoids, corticosteroids, elastase inhibitors, anti-fungals, oncology therapies, anti-emetics, analgesics, cardiovascular agents, anti-inflammatory agents, such as NSAIDs and COX-2 inhibitors, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives (hypnotics and neuroleptics), astringents, alpha-adrenergic receptor blocking agents, beta-adrenoceptor blocking agents, blood products and substitutes, cardiac inotropic agents, contrast media, corticosteroids, cough suppressants (expectorants and mucolytics), diagnostic agents, diagnostic imaging agents, diuretics, dopaminergics (antiparkinsonian agents), haemostatics, immunological agents, lipid regulating agents, muscle relaxants, parasympathomimetics, parathyroid calcitonin and biphosphonates, prostaglandins, radio- pharmaceuticals, sex hormones (including steroids), anti-allergic agents, stimulants and anoretics, sympathomimetics, thyroid agents, vasodilators, and xanthines.

A description of these classes of active agents and a listing of species within each class can be found in Martindale's The Extra Pharmacopoeia, 31st Edition (The Pharmaceutical Press, London, 1996), specifically incorporated by reference. The active agents are commercially available and/or can be prepared by techniques known in the art.

Exemplary non-sildenafil PDE5 inhibitors include, but are not limited to, vardenafil, tadalafil, TA-1790, UK-114542, Compound 14, EMD221829, EMR 62 203, T-1032, M-54033, M-54018, or E-4010. The term "PDE5 inhibitor" is used to mean any compound capable of inhibiting the enzyme phosphodiesterase type 5.

Exemplary nutraceuticals and dietary supplements are disclosed, for example, in Roberts et al., Nutraceuticals; The Complete Encyclopedia of Supplements, Herbs, Vitamins, and Healing Foods (American Nutraceutical Association, 2001), which is specifically incorporated by reference. Dietary supplements and nutraceuticals are also disclosed in Physicians' Desk Reference for Nutritional Supplements, 1 st Ed. (2001) and The Physicians' Desk Reference for Herbal Medicines, 1 st Ed. (2001), both of which are also incorporated by reference. A nutraceutical or dietary supplement, also known as phytochemicals or functional foods, is generally any one of a class of dietary supplements, vitamins, minerals, herbs, or healing foods that have medical or pharmaceutical effects on the body. Exemplary nutraceuticals or dietary supplements include, but are not limited to, lutein, folic acid, fatty acids (e.g., DHA and ARA), fruit and vegetable extracts, vitamin and mineral supplements, phosphatidylserine, lipoic acid, melatonin, glucosamine/chondroitin, Aloe Vera, Guggul, glutamine, amino acids (e.g., arginine, iso-leucine, leucine, lysine, methionine, phenylanine, threonine, tryptophan, and valine), green tea, lycopene, whole foods, food additives, herbs, phytonutrients, antioxidants, flavonoid constituents of fruits, evening primrose oil, flax seeds, fish and marine animal oils, and probiotics. Nutraceuticals and dietary supplements also include bio-engineered foods genetically engineered to have a desired property, also known as "pharmafoods."

Preferred combination therapies comprise a composition useful in methods of the invention with one or more active agents selected from alpha-adrenergic receptor blocking agents, such as yohimbine, delaquamine, phenotolamine and doxazosin, prostaglandins and prostoglandin analogs such as alprostadil and misoprostol, testosterone, anti-depressants such as trazodone, L-arginine, apomorphine, NO donors, and central nervous system stimulants.

Particularly preferred herbs and nutraceuticals of the invention are those known to be involved in biosynthesis/mediation of NO and cGMP, such as L-arginine and those known to be enhance sexual health and well-being, such as yohimbine, *Cornus officinalis, Cinnamomum aromaticum, Panax ginseng,* and *Pulsatilla pratensis.*

The compound to be administered in combination with a nanoparticulate sildenafil free base composition of the invention can be formulated separately from the sildenafil free base composition or co-formulated with the sildenafil free base composition. Where a sildenafil free base composition is co-formulated with a second active agent, the second active agent can be formulated in any suitable manner, such as immediate-release, rapid-onset, sustained-release, or dual-release form.

### 3. Surface Stabilizers

The choice of one or more surface stabilizers for nanoparticulate sildenafil free base is non-trivial and required extensive experimentation to realize a desirable formulation.

Combinations of more than one surface stabilizer can be used in the invention. Useful surface stabilizers which can be employed in the invention include, but are not limited to, known organic and inorganic pharmaceutical excipients. Such excipients include various polymers, low molecular weight oligomers, natural products, and surfactants. Surface stabilizers include nonionic, cationic, ionic, and zwitterionic compounds.

Representative examples of surface stabilizers include, but are not limited to, hydroxypropyl methylcellulose, hydroxypropylcellulose, polyvinylpyrrolidone, sodium lauryl sulfate, dioctylsulfosuccinate, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e.g*., macrogol ethers such as cetomacrogol 1000), polyoxyethylene castor oil derivatives, polyoxyethylene sorbitan fatty acid esters (*e.g*., the commercially available Tweens^{®} such as *e.g*., Tween 20^{®} and Tween 80^{®} (ICI Speciality Chemicals)); polyethylene glycols (*e.g*., Carbowaxs 3550^{®} and 934^{®} (Union Carbide)); polyoxyethylene stearates, colloidal silicon dioxide, phosphates, carboxymethylcellulose calcium, carboxymethylcellulose sodium, methylcellulose, hydroxyethylcellulose, hydroxypropylmethylcellulose phthalate, noncrystalline cellulose, magnesium aluminium silicate, triethanolamine, polyvinyl alcohol (PVA), 4-(1,1,3,3-tetramethylbutyl)-phenol polymer with ethylene oxide and formaldehyde (also known as tyloxapol, superione, and triton), poloxamers (*e.g*., Pluronics F68^{®} and F108^{®}, which are block copolymers of ethylene oxide and propylene oxide); poloxamines (*e.g.*, Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.)); Tetronic 1508^{®} (T-1508, a poloxamine) (BASF Wyandotte Corporation), Tritons X-200^{®}, which is an alkyl aryl polyether sulfonate (Rohm and Haas); Crodestas F-110^{®}, which is a mixture of sucrose stearate and sucrose distearate (Croda Inc.); p-isononylphenoxypoly-(glycidol), also known as Olin-IOG^{®} or Surfactant 10-G^{®} (Olin Chemicals, Stamford, CT); Crodestas SL-40^{®} (Croda, Inc.); and SA9OHCO, which is C₁₈H₃₇CH₂C(O)N(CH₃)CH₂(CHOH)₄(CH₂OH)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, and the like.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quartemary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C₁₂₋₁₅ dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts and dialkyl-dimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT 336^{™}), POLYQUAT 10^{™} (polyquaternium 10; Buckman Laboratories, TN), tetrabutylammonium bromide, benzyl trimethylammonium bromide, choline esters (such as choline esters of fatty acids), benzalkonium chloride, stearalkonium chloride compounds (such as stearyltrimonium chloride and Di-stearyldimonium chloride), cetyl pyridinium bromide or chloride, halide salts of quaternized polyoxyethylalkylamines, MIRAPOL^{™} (quaternized ammonium salt polymers) and ALKAQUAT^{™} (benzalkonium chloride) (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Nonpolymeric surface stabilizers are any nonpolymeric compound, such benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quaternary ammonium compounds of the formula NR₁R₂R₃R₄⁽⁺⁾. For compounds of the formula NR₁R₂R₃R₄⁽⁺⁾:
(i) none of R₁-R₄ are CH₃;
(ii) one of R₁-R₄ is CH₃;
(iii) three of R₁-R₄ are CH₃;
(iv) all of R₁-R₄ are CH₃;
(v) two of R₁-R₄ are CH₃, one of R₁-R₄ is C₆H₅CH₂, and one of R₁-R₄ is an alkyl chain of seven carbon atoms or less;
(vi) two of R₁-R₄ are CH₃, one of R₁-R₄ is C₆H₅CH₂, and one of R₁-R₄ is an alkyl chain of nineteen carbon atoms or more;
(vii) two of R₁-R₄ are CH₃ and one of R₁-R₄ is the group C₆H₅(CH₂)ₙ, where n>1;
(viii) two of R₁-R₄ are CH₃, one of R₁-R₄ is C₆H₅CH₂, and one of R₁-R₄ comprises at least one heteroatom;
(ix) two of R₁-R₄ are CH₃, one of R₁-R₄ is C₆H₅CH₂, and one of R₁-R₄ comprises at least one halogen;
(x) two of R₁-R₄ are CH₃, one of R₁-R₄ is C₆H₅CH₂, and one of R₁-R₄ comprises at least one cyclic fragment;
(xi) two of R₁-R₄ are CH₃ and one of R₁-R₄ is a phenyl ring; or
(xii) two of R₁-R₄ are CH₃ and two of R₁-R₄ are purely aliphatic fragments.

Such compounds include, but are not limited to, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride(Quaternium-14), Quaternium-22, Quatemium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCl, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

The surface stabilizers are commercially available and/or can be prepared by techniques known in the art. Many are described in detail in the *Handbook of Pharmaceutical Excipients,* published jointly by the American Pharmaceutical Association and The Pharmaceutical Society of Great Britain (The Pharmaceutical Press, 2000), specifically incorporated by reference.

### 4. Other Pharmaceutical Excipients

Pharmaceutical sildenafil free base compositions according to the invention may also comprise one or more binding agents, filling agents, lubricating agents, suspending agents, sweeteners, flavoring agents, preservatives, buffers, wetting agents, disintegrants, effervescent agents, and other excipients. Such excipients are known in the art.

Examples of filling agents are lactose monohydrate, lactose anhydrous, and various starches; examples of binding agents are various celluloses and cross-linked polyvinylpyrrolidone, microcrystalline cellulose, such as Avicel^{®} PH101 and Avicel^{®} PH102, microcrystalline cellulose, and silicified microcrystalline cellulose (ProSolv SMCC^{™}).

Suitable lubricants, including agents that act on the flowability of a powder to be compressed, are colloidal silicon dioxide, such as Aerosil^{®} 200, talc, stearic acid, magnesium stearate, calcium stearate, and silica gel.

Examples of sweeteners are any natural or artificial sweetener, such as sucrose, xylitol, sodium saccharin, cyclamate, aspartame, and acsulfame. Examples of flavoring agents are Magnasweet^{®} (trademark of MAFCO), bubble gum flavor, mint flavor, and fruit flavors, and the like.

Examples of preservatives are potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quartemary compounds such as benzalkonium chloride.

Suitable diluents include pharmaceutically acceptable inert fillers, such as microcrystalline cellulose, lactose, dibasic calcium phosphate, saccharides, and/or mixtures of any of the foregoing. Examples of diluents include microcrystalline cellulose, such as Avicel^{®} PH101 and Avicel^{®} PH102; lactose such as lactose monohydrate, lactose anhydrous, and Pharmatose^{®} DCL21; dibasic calcium phosphate such as Emcompress^{®}; mannitol; starch; sorbitol; sucrose; and glucose.

Suitable disintegrants include lightly crosslinked polyvinyl pyrrolidone, corn starch, potato starch, maize starch, and modified starches, croscarmellose sodium, cross-povidone, sodium starch glycolate, and mixtures thereof.

Examples of effervescent agents are effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

### 5. Nanoparticulate Sildenafil Free Base and Non-Sildenafil Active Agent Particle Size

The compositions of the invention comprise sildenafil free base particles which have an effective average particle size of less than about 2000 nm (*i*.*e*., 2 microns). In other embodiments of the invention, the sildenafil free base particles have an effective average particle size of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

If the composition additionally comprises one or more nanoparticulate non-sildenafil free base active agents, then such active agents may have an effective average particle size of less than about 2000 nm, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

By "an effective average particle size of less than about 2000 nm" it is meant that at least 50% of the sildenafil free base or non-sildenafil free base active agent particles have a particle size, by weight, of less than the effective average particle size when measured by the above-noted techniques, *e.g*., 50% of the particles have a size, by weight, of less than about 2 microns (or less than about 1900 nm, less than about 1800 nm, *etc*.). In other embodiments of the invention, at least about 70%, at least about 90%, at least about 95%, or at least about 99%, by weight, of the sildenafil free base or non-sildenafil free base active agent particles have a particle size less than the effective average, *e.g*., less than about 2000 nm, less than about 1900 nm, less than about 1800 nm, *etc.*

If the nanoparticulate sildenafil free base is combined with a non-nanoparticulate active agent composition, then such a composition is either solubilized or has an effective average particle size of greater than about 2 microns. By "an effective average particle size of greater than about 2 microns" it is meant that at least 50% of the active agent particles have a particle size of greater than about 2 microns, by weight, when measured by the above-noted techniques. In other embodiments of the invention, at least about 70%, at least about 90%, at least about 95%, or at least about 99% of the active agent particles have a particle size greater than about 2 microns.

In the present invention, the value for D50 of a nanoparticulate sildenafil free base composition is the particle size below which 50% of the sildenafil free base particles fall, by weight. Similarly, D90 and D95 are the particle sizes below which 90% and 95%, respectively, of the sildenafil free base particles fall, by weight.

### 6. Concentration of Nanoparticulate Sildenafil Free Base and Surface Stabilizer

The relative amounts of nanoparticulate sildenafil free base and one or more surface stabilizers can vary widely. The optimal amount of the individual components can depend, for example, upon the hydrophilic lipophilic balance (HLB), melting point, and the surface tension of water solutions of the surface stabilizer, *etc*.

. The concentration of sildenafil free base can vary from about 99.5% to about 0.001 %, from about 95% to about 0.1%, or from about 90% to about 0.5%, by weight, based on the total combined dry weight of sildenafil free base and at least one surface stabilizer, not including other excipients.

The concentration of at least one surface stabilizer can vary from about 0.5% to about 99.999%, from about 5.0% to about 99.9%, or from about 10% to about 99.5%, by weight, based on the total combined dry weight of sildenafil free base and at least one surface stabilizer, not including other excipients.

### C. Methods of Making Nanoparticulate Sildenafil Free Base Compositions

The sildenafil free base compositions of the invention can be made using, for example, milling, homogenization, or precipitation techniques. Exemplary methods of making nanoparticulate compositions are described in U.S. Patent No. 5,145,684.

Methods of making nanoparticulate active agent compositions are also described in U.S. Patent No. 5,518,187 for "Method of Grinding Pharmaceutical Substances;" U.S. Patent No. 5,718,388 for "Continuous Method of Grinding Pharmaceutical Substances;" U.S. Patent No. 5,862,999 for "Method of Grinding Pharmaceutical Substances;" U.S. Patent No. 5,665,331 for "Co-Microprecipitation of Nanoparticulate Pharmaceutical Agents with Crystal Growth Modifiers;" U.S. Patent No. 5,662,883 for "Co-Microprecipitation of Nanoparticulate Pharmaceutical Agents with Crystal Growth Modifiers;" U.S. Patent No. 5,560,932 for "Microprecipitation of Nanoparticulate Pharmaceutical Agents;" U.S. Patent No. 5,543,133 for "Process of Preparing X-Ray Contrast Compositions Containing Nanoparticles;" U.S. Patent No. 5,534,270 for "Method of Preparing Stable Drug Nanoparticles;" U.S. Patent No. 5,510,118 for "Process of Preparing Therapeutic Compositions Containing Nanoparticles;" and U.S. Patent No. 5,470,583 for "Method of Preparing Nanoparticle Compositions Containing Charged Phospholipids to Reduce Aggregation," all of which are specifically incorporated by reference.

One or more non-sildenafil free base active agents can be reduced in size at the same time as sildenafil free base, to produce a nanoparticulate sildenafil free base and nanoparticulate non-sildenafil free base active agent composition. A non-sildenafil free base active agent, which is either non-nanoparticulate or nanoparticulate sized, can also be added to the nanoparticulate sildenafil free base composition after particle size reduction.

In yet another embodiment of the invention, nanoparticulate sildenafil free base compositions of the invention can be made in which the formulation comprises multiple nanoparticulate sildenafil free base compositions, each of which has a different effective average particle size. Such a composition can be made by preparing the individual nanoparticulate sildenafil free base compositions using, for example, milling, precipitation, or homogenization techniques, followed by combining the different compositions to prepare a single dosage form.

The nanoparticulate sildenafil free base compositions can be utilized in solid or liquid dosage formulations, such as liquid dispersions, gels, aerosols, ointments, creams, controlled release formulations, fast melt formulations, lyophilized formulations, tablets, capsules, delayed release formulations, extended release formulations, pulsatile release formulations, mixed immediate release and controlled release formulations, etc. Aerosols, including both nasal sprays and pulmonary aerosols, are expected to be particularly useful for delivering nanoparticulate sildenafil free base compositions having a fast onset of activity.

### 1. Milling to Obtain Nanoparticulate Sildenafil Free Base Compositions

Milling sildenafil free base to obtain a nanoparticulate composition comprises dispersing particles of sildenafil free base in a liquid dispersion media in which sildenafil free base is poorly soluble, followed by applying mechanical means in the presence of rigid grinding media to reduce the particle size of sildenafil free base to the desired effective average particle size. The dispersion media can be, for example, water, safflower oil, ethanol, t-butanol, glycerin, polyethylene glycol (PEG), hexane, or glycol. Water is a preferred dispersion media.

The sildenafil free base particles are preferably reduced in size in the presence of at least one surface stabilizer. Alternatively, the sildenafil free base particles can be contacted with one or more surface stabilizers after attrition. Other compounds, such as a diluent, can be added to the sildenafil free base/surface stabilizer composition during the particle size reduction process. Dispersions can be manufactured continuously or in a batch mode.

### 2. Precipitation to Obtain Nanoparticulate Sildenafil Free Base Compositions

Another method of forming the desired nanoparticulate sildenafil free base composition is by microprecipitation. This is a method of preparing stable dispersions of poorly soluble active agents in the presence of one or more surface stabilizers and one or more colloid stability enhancing surface active agents free of any trace toxic solvents or solubilized heavy metal impurities. Such a method comprises, for example: (1) dissolving sildenafil free base in a suitable solvent; (2) adding the formulation from step (1) to a solution comprising at least one surface stabilizer, and (3) precipitating the formulation from step (2) using an appropriate non-solvent. The method can be followed by removal of any formed salt, if present, by dialysis or diafiltration and concentration of the resultant nanoparticulate sildenafil free base dispersion by conventional means.

### 3. Homogenization to Obtain Nanoparticulate Sildenafil Free Base Compositions

Exemplary homogenization methods of preparing nanoparticulate active agent compositions are described in U.S. Patent No. 5,510,118, for "Process of Preparing Therapeutic Compositions Containing Nanoparticles." Such a method comprises dispersing sildenafil free base particles in a liquid dispersion media, followed by subjecting the dispersion to homogenization to reduce the particle size of sildenafil free base to the desired effective average particle size. The sildenafil free base particles are preferably reduced in size in the presence of at least one surface stabilizer. Alternatively, the sildenafil free base particles can be contacted with one or more surface stabilizers either before or after attrition. Other compounds, such as a diluent, can be added to the sildenafil free base/surface stabilizer composition either before, during, or after the particle size reduction process. Dispersions can be manufactured continuously or in a batch mode.

### D. Methods of Using the Sildenafil Free Base Compositions of the Invention

The present invention provides a method of rapidly increasing the plasma levels of sildenafil in a subject. Such a method comprises administering to a subject an effective amount of a nanoparticulate sildenafil free base composition of the invention.

The sildenafil free base compositions of the invention can be administered to a subject via any conventional means including, but not limited to, orally, rectally, ocularly, parenterally (*e.g*., intravenous, intramuscular, or subcutaneous), intracisternally, pulmonary, intravaginally, intraperitoneally, locally (*e.g*., powders, ointments or drops), or as a buccal or nasal spray. As used herein, the term "subject" is used to mean an animal, preferably a mammal, including a human or non-human. The terms patient and subject may be used interchangeably.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents, or vehicles including water, ethanol, polyols (propyleneglycol, polyethylene-glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants.

The nanoparticulate sildenafil free base compositions may also contain adjuvants such as preserving, wetting, emulsifying, and dispensing agents. Prevention of the growth of microorganisms can be ensured by various antibacterial and antifungal agents, such as parabens, chlorobutanol, phenol, sorbic acid, and the like. It may also be desirable to include isotonic agents, such as sugars, sodium chloride, and the like. Prolonged absorption of the injectable pharmaceutical form can be brought about by the use of agents delaying absorption, such as aluminum monostearate and gelatin.

Solid dosage forms for oral administration include, but are not limited to, capsules, tablets, pills, powders, and granules. In such solid dosage forms, sildenafil free base is admixed with at least one of the following: (a) one or more inert excipients (or carriers), such as sodium citrate or dicalcium phosphate; (b) fillers or extenders, such as starches, lactose, sucrose, glucose, mannitol, and silicic acid; (c) binders, such as carboxymethylcellulose, alignates, gelatin, polyvinylpyrrolidone, sucrose, and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as cetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration include pharmaceutically acceptable emulsions, solutions, suspensions, syrups, and elixirs. In addition to sildenafil free base, the liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like.

Besides such inert diluents, the sildenafil free base composition can also include adjuvants, such as wetting agents, emulsifying and suspending agents, sweetening, flavoring, and perfuming agents.

One of ordinary skill will appreciate that effective amounts sildenafil free base can be determined empirically and can be employed in pure form or, where such forms exist, in pharmaceutically acceptable salt, ester, or prodrug form. Actual dosage levels of sildenafil free base in the nanoparticulate compositions of the invention may be varied to obtain an amount of sildenafil free base that is effective to obtain a desired therapeutic response for a particular composition and method of administration. The selected dosage level, therefore, depends upon the desired therapeutic effect, the route of administration, the potency of the administered sildenafil free base, the desired duration of treatment, and other factors.

Dosage unit compositions may contain such amounts of such sub-multiples thereof as may be used to make up the daily dose. It will be understood, however, that the specific dose level for any particular patient will depend upon a variety of factors: the type and degree of the cellular or physiological response to be achieved; activity of the specific agent or composition employed; the specific agents or composition employed; the age, body weight, general health, sex, and diet of the patient; the time of administration, route of administration, and rate of excretion of the agent; the duration of the treatment; drugs used in combination or coincidental with the specific agent; and like factors well known in the medical arts.

### 1. Use of Additional Active Agents

The methods of the invention also encompass administering a nanoparticulate sildenafil free base composition of the invention in combination with one or more non-sildenafil active agents, in either a conventional or nanoparticulate form. These additional active agents are discussed above.

### 2. Treatment Applications

The sildenafil free base compositions of the invention are useful in treating and/or preventing, among other conditions, mammalian sexual disorders. In particular, the compositions are of value in the treatment of mammalian sexual dysfunctions such as male erectile dysfunction, impotence, female sexual dysfunction, clitoral dysfunction, female hypoactive sexual desire disorder, female sexual arousal disorder, female sexual pain disorder or female sexual orgasmic dysfunction, as well as sexual dysfunction due to spinal cord injury.

The sildenafil free base compositions of the invention are useful for treating other medical conditions for which a PDE5 inhibitor is indicated. Such conditions include but are not limited to premature labor, dysmenorrhea, benign prostatic hyperplasia, bladder outlet obstruction, incontinence, stable, unstable and variant (Prinzmetal) angina, hypertension, pulmonary hypertension, chronic obstructive pulmonary disease, coronary artery disease, congestive heart failure, atherosclerosis, conditions of reduced blood vessel patency, e.g. post-percutaneous transluminal coronary angioplasty (post-PTCA), peripheral vascular disease, stroke, nitrate induced tolerance, bronchitis, allergic asthma, chronic asthma, allergic rhinitis, glaucoma and diseases characterized by disorders of gut motility, *e.g*. irritable bowel syndrome and diabetic gastroparesis.

Further medical conditions for which a PDE5 inhibitor is indicated, and for which treatment with the sildenafil free base compositions of the present invention may be useful, include pre-eclampsia, Kawasaki's syndrome, nitrate tolerance, multiple sclerosis, diabetic nephropathy, peripheral diabetic neuropathy, Alzheimer's disease, acute respiratory failure, psoriasis, skin necrosis, cancer, metastasis, baldness, nutcracker oesophagus, anal fissure, hemorrhoids and hypoxic vasoconstriction.

The following examples are given to illustrate the present invention. It should be understood, however, that the invention is not to be limited to the specific conditions or details described in these examples. Throughout the specification, any and all references to a publicly available document, including a U.S. patent, are specifically incorporated by reference.

### Example 1

The purpose of this example was to prepare a composition of nanoparticulate sildenafil free base.

Sildenafil free base was prepared from sildenafil citrate as follows. 5 grams of sildenafil citrate (Cipla) was dissolved into approximately 1500 mL of DI water. The sildenfil citrate solution was mixed for a 6 to 12 hour time period. Once the drug was sufficiently dissolved, any remaining undissolved drug was filtered off using 1 micron glass fiber filter paper. The substrate was collected into a large filtration flask and then transferred into a large beaker and placed on a magnetic stir plate. 1N NaOH was titrated in approximately 7 mL increments to the mixing solution until the sildenafil free base precipitate was formed and the solution was approximately pH 10. The sildenafil free base precipitate was then collected via filtration using 1 micron glass fiber filter paper. The beaker was rinsed with DI water to collect any residual precipitate and added to the filter paper. The filter paper and the filtration funnel were place in an oven set at 40°C and dried over night. Once dry the sildenafil free base was harvested from the filter paper into a tared vial.

4.0 g of the sildenafil free base was added to a solution containing hydroxypropylmethylcellulose (HPMC, Hypromellose, USP, Pharmacoat® 603, substitution type 2910, 3 cp) (Shin Etsu Chemical Co., Ltd.) (0.8 g), docusate sodium (DOSS) (Cytec Industries, Inc.) (0.04 g), and water (75.16 g). This mixture was then milled for 90 minutes in a DYNO-Mill KDL (Willy A. Bachofen AG, Maschinenfabrik, Basel, Switzerland) at 4200 RPM with PM-500 polymeric media.

The final mean (weight average) particle size of the sildenafil particles was 281 nm, with a D50 of 274 nm, a D90 of 371 nm, and a D95 of 407 nm, as measured on a Horiba LA-910 particle size analyzer (Horiba Instruments, Irvine, CA).

This example demonstrates the successful preparation of a dispersion of nanoparticulate sildenafil free base.

### Example 2

The purpose of this example was to prepare a lyophilized wafer dosage form of the nanoparticulate sildenafil free base dispersion prepared in Example 1.

30 grams of the nanoparticulate sildenafil free base dispersion from Example 1 was added to 3.0 grams mannitol USP/NF (Spectrum) and 1.5 grams pullulan (Hayashibara).

A wafer tray was then filled by adding 0.5 grams of the diluted sildenafil free base dispersion to each 0.5 cc well and the wafer tray was then placed in a lyophilizer for 48 hours to produce the final lyophilized wafer dosage form.

The sildenafil free base particle size in the lyophilized wafers appeared stable. After reconstitution in an aqueous media, the mean particle size (by weight) of sildenafil free base was 306 nm, with a D50 of 283 nm, a D90 of 443 nm, and a D95 of 522 nm.

A summary of the sildenafil free base particle size data from Examples 1 and 2 is shown in the following table.

| **TABLE 1** | | | | |
|---|---|---|---|---|
| **Composition** | **Mean Particle Size (nm)** | **D50 (nm)** | **D90 (nm)** | **D95 (nm)** |
| Sildenafil Free Base + HPMC + DOSS | 281 | 274 | 371 | 407 |
| Sildenafil Free Base + HPMC + Doss: Reconstituted in Water | 306 | 283 | 443 | 522 |

This example demonstrates the successful preparation of a solid dosage form of a dispersion of nanoparticulate sildenafil free base. Moreover, this example demonstrates the successful preparation of a solid dosage form of a dispersion of nanoparticulate sildenafil free base in which the sildenafil free base particles redisperse upon reconstitution in an aqueous media such that the particle size of sildenafil is substantially equivalent to the particle size prior to incorporation into a solid dosage form.

### Example 3

The purpose of this example was to prepare a composition of nanoparticulate sildenafil citrate.

To identify the best surface stabilizer for sildenafil citrate, the compound was screened in a NanoMill® (Elan Drug Delivery, Inc.) (*see e.g*., WO 00/72973 for "Small-Scale Mill and Method Thereof") with the following surface stabilizers: hydroxypropylmethylcellulose (HPMC), hydroxypropylcellulose (HPC-SL), polyvinylpyrrolidone (PVP K29\32), and Plasdone® S630. The HPMC and HPC-SL formulations looked the best under a microscope, with the HPC-SL looking the better of the two. Thus, while HPMC was used in the nanoparticulate formulation of sildenafil free base, HPC-SL was chosen as the best surface stabilizer for a nanoparticulate formulation of sildenafil citrate.

Sildenafil citrate (Cipla) (4.0 g) was added to a solution containing hydroxypropylcellulose (HPC-SL) (Nisso) (0.8 g) and water (75.2 g). The mixture was then milled for 90 minutes in a DYNO-Mill KDL (Willy A. Bachofen AG, Maschinenfabrik, Basel, Switzerland) at 4200 RPM with PM-500 polymeric media.

Small (nanoparticulate), well dispersed sildenafil citrate particles were observed in the milled sildenafil dispersion on a Leica microscope. (Because sildenafil citrate is very soluble, and because only a small volume of nanoparticulate sildenafil citrate dispersion was available, it was not possible to specifically measure the sildenafil citrate particle size, at the dilute concentration of sildenfil citrate present in the nanoparticulate dispersion, using, *e.g*., Horiba LA-910 particle size analyzer. Particle size of nanoparticulate sildenafil citrate could potentially be measured using, e.g., a Horiba particle size analyzer, if a saturated dispersion of sildenafil citrate, or a larger volume of dispersion to be measured, was used.)

### Example 4

The purpose of this example was to prepare a lyophilized wafer dosage form of the nanoparticulate sildenafil citrate composition prepared in Example 3.

42 grams of the nanoparticulate sildenafil citrate dispersion of Example 3 were added to 4.2 grams mannitol USP/NF (Spectrum) and 2.2 grams pullulan (Hayashibara).

A wafer tray was filled by adding 0.5 grams of the diluted nanoparticulate sildenafil citrate dispersion to each 0.5 cc well. The wafer tray was then placed in a lyophilizer for 48 hours to produce the final lyophilized wafer dosage form.

The particle size of the reconstituted sildenafil citrate lyophilized dosage form was not measured, for the same reasons given above in Example 3. However, the presence of nanoparticles of sildenafil citrate, following reconstitution in an aqueous media, was optically confirmed via a Leica microscope.

### Example 5

The purpose of this example was to evaluate the *in vivo* pharmacokinetics, under fed and fasted conditions, for the nanoparticulate sildenafil free base and nanoparticulate sildenafil citrate dosage forms of Examples 2 and 4, respectively, and Viagra®, which is a non-nanoparticulate sildenfil citrate dosage form.

### Dog Study Protocol

Eight male and four female Beagle dogs were used in a study having five different phases. The three sildenafil formulations evaluated in the study are summarized in Table 2. Preparation of the samples is described above in Examples 1-4.

| **TABLE 2** | | | |
|---|---|---|---|
| **Summary of Sildenafil Formulations** | | | |
| **Sample** | **Components** | **Dosage Form** | **Particle Size of Sildenafil** |
| Formulation #1 (Example 1) | sildenafil free base, HPMC, DOSS, | dispersion | 281 nm |
| Formulation #2 (Example 2) | sildenafil free base, HPMC, DOSS, mannitol, pullulan | wafer; 25 mg | 306 nm (redispersed) |
| Formulation #3 (Example 3) | sildenafil citrate, HPC-SL | dispersion | Could not specifically measure particle size; Visually confirmed via microscope the presence of nanosized particles |
| Formulation #4 (Example 4) | sildenafil citrate, HPC-SL, mannitol, pullulan | wafer; 25 mg | Could not specifically measure particle size; Visually confirmed via microscope the presence of redispersed nanosized particles |
| Formulation #5 | sildenafil citrate (Viagra® tablets; Pfizer Labs) | tablet; 25 mg | Microparticulate |

In all phases of the study, two hours before dosing each dog received 22.5 mg of Zantac® (15 g/mL Zantac® syrup). Zantac® was administered to reduce the highly acidic conditions present in dog stomachs. The reduced acidity correlates more accurately to human stomach conditions. In addition, sildenafil citrate and sildenafil free base solubilize in highly acidic conditions. If such solubilization occurred in the stomach, the drugs would then likely precipitate into larger particles in the intestine, thus negating the benefits of formulating the drugs into a nanoparticulate dosage form.

### Phase 1 of the Study (Fed Conditions):

In Phase 1, all dogs were fed. Six dogs (4 males and 2 females) received Formulation #2, a 25 mg lyophilized wafer of a dispersion of nanoparticulate sildenafil free base, and six dogs (4 males and 2 females) receive a 25 mg tablet of Viagra® (micronized sildenafil citrate).

### Phase 2 of the Study (Fed Conditions):

After a four day washout period, Phase 2 was initiated in fed dogs. The six dogs which received Formulation #2 (25 mg lyophilized wafer of a dispersion of nanoparticulate sildenafil free base) in Phase 1 now received a 25 mg tablet of Viagra®, and the other 6 dogs received Formulation #4, a 25 mg lyophilized wafer of a dispersion of nanoparticulate sildenafil citrate.

### Phase 3 of the Study (Fasted Conditions):

After another four-day washout period, the dosing regimen used in Phase 1 was used in Phase 3. Phase 3 dogs were fasted overnight and for four hours post dose.

### Phase 4 of the Study (Fasted Conditions):

After another four days washout period, the dosing regimen used in Phase 2 was used in Phase 4. Phase 4 dogs were fasted overnight and for four hours post dose.

### Phase 5 of the Study (Fasted Conditions):

Finally, after another four day washout period, the Phase 5 dogs were fasted overnight and for four hours postdose. The first six dogs received Formulation #4 (25 mg lyophilized wafer of a dispersion of nanoparticulate sildenafil citrate), while the second six dogs received Formulation #2 (25 mg lyophilized wafer of a dispersion of nanoparticulate sildenafil free base).

Blood samples (approximately 1.0 mL) were collected at specified time points (pre-dose, 0.08, 0.17, 0.25, 0.33, 0.5, 0.75, 1, 1.5, 2, 4, 6, and 8 hours postdose) into tubes containing sodium heparin. The samples were

A summary of the five phase study is shown below in Table 3.

| **TABLE 3** | | | | | | |
|---|---|---|---|---|---|---|
| **Summary of the Five Phase Study** | | | | | | |
| **Phase** | **Compound** | **Fed/ Fasted** | **Number of Males/ Females** | **Dose Route** | **Dose Level (mg)** | **Matrix Collected** |
| 1 | Nano Sildenafil Free Base (#2) /Viagra® | Fed | 4/2;4/2 | Wafer/ Tablet | 25 | Blood^{a} |
| 2 | Viagra®/ Nano Sildenafil Citrate (#4) | Fed | 4/2;4/2 | Tablet/ Wafer | 25 | Blood^{a} |
| 3 | Nano Sildenafil Free Base (#2) /Viagra® | Fasted | 4/2;4/2 | Wafer/ Tablet | 25 | Blood^{a} |
| 4 | Viagra®/ Nano Sildenafil Citrate (#4) | Fasted | 4/2;4/2 | Tablet/ Wafer | 25 | Blood^{a} |
| 5 | Nano Sildenafil Citrate (#4)/ Nano Sildenafil Free Base (#2) | Fasted | 4/2;4/2 | Wafer/ Wafer | 25 | Blood^{a} |

The blood samples were then evaluated to determine the Cₘₐₓ, Tₘₐₓ, AUCₗₐₛₜ, and AUC_{inf} for Viagra® given under fed and fasted conditions, and for the nanoparticulate sildenafil free base (Formulation #2) and nanoparticulate sildenafil citrate (Formulation #4) compositions, given under fed and fasted conditions. The results are shown below in Table 4 and in Figures 1 and 2.

| **TABLE 4** | | | | |
|---|---|---|---|---|
| **Pharmacokinetic Results of the Dog Study** | | | | |
| | **Cₘₐₓ** | **Tₘₐₓ** | **AUCₗₐₛₜ** | **AUC_{inf}** |
| | **(ng/mL)** | **(hours)** | **(ng/mL.h)** | **(ng/mL.h)** |
| Viagra® FASTED | 234.6 | 1.85 | 1124.4 | 2514 |
| Viagra®, FED | 303.4 | 0.74 | 1166.25 | 1749.6 |
| Formulation #2 | 285.3 | 1.08 | 1332.1 | 2127.8 |
| (nanoparticulate sildenafil free base) | | | | |
| FASTED | | | | |
| Formulation #2 | 319.7 | 1.125 | 1329.3 | 2105.3 |
| (nanoparticulate sildenafil free base) | | | | |
| FED | | | | |
| Formulation #4 | 319.7 | 0.6 | 1035.9 | 1335.3 |
| (nanoparticulate sildenafil citrate) | | | | |

| **TABLE 4** | | | | |
|---|---|---|---|---|
| **Pharmacokinetic Results of the Dog Study** | | | | |
| | **Cₘₐₓ** | **Tₘₐₓ** | **AUCₗₐₛₜ** | **AUC_{inf}** |
| | **(ng/mL)** | **(hours)** | **(ng/mL.h)** | **(ng/mL.h)** |
| FASTED | | | | |
| Formulation #4 | 162.9 | 1.25 | 647.6 | 897.8 |
| (nanoparticulate sildenafil citrate) | | | | |
| FED | | | | |

Figure 1 shows the mean sildenafil concentration (ng/mL) for nanoparticulate sildenafil free base (Formulation #2), nanoparticulate sildenafil citrate (Formulation #4), and Viagra®, given under fed and fasted conditions, over a 2 hour time period. Figure 2 shows the mean sildenafil concentration (ng/mL) for Formulations 2, 4, and Viagra®, given under fed and fasted conditions, over an 8 hour time period.

The results show that the nanoparticulate sildenafil free base composition of the invention demonstrates substantially similar AUC profiles when administered under fed and fasted conditions.

This is significant, as it means that there is no substantial difference in the quantity of sildenafil free base absorbed or the rate of sildenafil free base absorption when the nanoparticulate sildenafil free base composition is administered in the fed versus the fasted state.

Viagra® administered under fed and fasted conditions exhibited an AUC_{inf} of 2514 ng/mL·h and 1749.6 ng/mL·h, respectively - a difference of 764.4 ng/mL·h or ~ 30%. The nanoparticulate sildenafil citrate composition administered under fed and fasted conditions exhibited an AUC_{inf} of 1335.2 ng/mL·h and 897.8 ng/mL-h, respectively - a difference of 437.4 ng/mL·h or ~ 33%. In surprising contrast to these results, the nanoparticulate sildenafil free base composition, administered under fed and fasted conditions, exhibited an AUC_{inf} of 2127.8 ng/mL·h and 2105.3 ng/mL·h, respectively - a difference of 22.5 ng/mL·h or ~1%.

The AUCₗₐₛₜ difference of 22.5 ng/mL·h for administration under fed and fasted conditions for the nanoparticulate sildenafil free base composition AUC_{inf} of 2127.8 ng/mL·h and 2105.3 ng/mL·h, respectively - a difference of 22.5 ng/mL·h or~1%.

The AUCₗₐₛₜ difference of 22.5 ng/mL·h for administration under fed and fasted conditions for the nanoparticulate sildenafil free base composition is almost *40 times* less than the fed/fasted variability observed with Viagra®, and over *19 times* less than the fed/fasted variability observed with the nanoparticulate sildenafil citrate composition.

Moreover, the Tₘₐₓ and Cₘₐₓ profiles for the nanoparticulate sildenafil free base composition were substantially similar when administered under fed and fasted conditions: - 11% difference for Cₘₐₓ and a 4% difference for Tₘₐₓ. In contrast, both the Viagra® and nanoparticulate sildenafil citrate compositions showed dramatically different results for Tₘₐₓ and Cₘₐₓ when the formulations were administered under fed as compared to fasted conditions: for Viagra®, a ~23% difference in Cₘₐₓ and 60% difference in Tₘₐₓ. and for the nanoparticulate sildenafil citrate composition, a 49% difference in Cₘₐₓ and 52% difference in Tₘₐₓ.

## Claims

1. A nanoparticulate sildenafil free base composition comprising:
(a) particles of sildenafil free base having an effective average particle size of less than about 2000 nm; and
(b) at least one surface stabilizer,
wherein the composition does not produce significantly different absorption levels when administered under fed as compared to fasting conditions.

2. A composition of claim 1, wherein the difference in the:
(a) AUC of the nanoparticulate sildenafil free base composition of the invention, when administered in the fed versus the fasted state, is selected from the group consisting of less than about 35%, less than about 30%, less than about 25%, less than about 20%, less than about 15%, less than about 10%, less than about 5%, less than about 3%, and less than about 1%; and/or
(b) Tₘₐₓ for the nanoparticulate sildenafil free base composition of the invention, when administered in the fed versus the fasted state, is less than about 60%, less than about 50%, less than about 40%, less than about 30%, less than about 20%, less than about 15%, less than about 10%, less than about 5%, and less than about 3%; and/or
(c) Cₘₐₓ for the nanoparticulate sildenafil free base composition of the invention, when administered in the fed versus the fasted state, is less than about 50%, less than about 40%, less than about 30%, less than about 20%, less than about 15%, less than about 10%, less than about 5%, and less than about 3%.

3. A composition of claim 1 or claim 2, wherein:
(a) the AUC of the composition is not significantly different following administration under fed as compared to fasting conditions; and/or
(b) the Tₘₐₓ of the composition is not significantly different following administration under fed as compared to fasting conditions; and/or
(c) the Cₘₐₓ of the composition is not significantly different following administration under fed as compared to fasting conditions.

4. A composition of any one of claims 1 to 3, wherein sildenafil free base is selected from the group consisting of a crystalline phase, an amorphous phase, a semi-crystalline phase, a semi-amorphous phase, and mixtures thereof.

5. A composition of any one of claims 1 to 4, wherein the effective average particle size of sildenafil free base is selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.

6. A composition of any one of claims 1 to 5, wherein the composition is formulated:
(a) for administration selected from the group consisting of oral, pulmonary, rectal, opthalmic, colonic, parenteral, intracisternal, intravaginal, intraperitoneal, local, buccal, nasal, and topical administration; and/or
(b) into a dosage form selected from the group consisting of liquid dispersions, gels, aerosols, ointments, creams, controlled release formulations, fast melt formulations, lyophilized formulations, tablets, capsules, delayed release formulations, extended release formulations, pulsatile release formulations, and mixed immediate release and controlled release formulations.

7. A composition of any one of claims 1 to 6, formulated into an aerosol or nasal spray and having a Tₘₐₓ which is less than that observed with a composition of non-nanoparticulate sildenafil.

8. A composition of any one of claims 1 to 7, wherein the composition further comprises one or more pharmaceutically acceptable excipients, carriers, or a combination thereof.

9. A composition of any one of claims 1 to 8, wherein:
(a) sildenaril free base is present in an amount selected from the group consisting of from about 99.5% to about 0.001%, from about 95% to about 0.1 %, and from about 90% to about 0.5%, by weight, based on the total combined dry weight of sildenafil free base and at least one surface stabilizer, not including other excipients; and/or
(b) at least one surface stabilizer is present in an amount selected from the group consisting of from about 0.5% to about 99.999%, from about 5.0% to about 99.9%, or from about 10% to about 99.5%, by weight, based on the total combined dry weight of the sildenafil free base and at least one surface stabilizer, not including other excipients.

10. A composition of any one of claims 1 to 9, comprising at least two surface stabilizers.

11. A composition of any one of claims 1 to 10, wherein the surface stabilizer is selected from the group consisting of a nonionic surface stabilizer, an anionic surface stabilizer, a cationic surface stabilizer, an ionic surface stabilizer, and a zwitterionic surface stabilizer.

12. A composition of any one of claims 1 to 11, wherein:
(a) at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hydroxypropyl methylcellulose, carboxymethylcellulose sodium, methylcellulose, hydroxyethylcellulose, hydroxypropylmethyl-cellulose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate, dialkylesters of sodium sulfosuccinic acid, sodium lauryl sulfate, alkyl aryl polyether sulfonates, mixtures of sucrose stearate and sucrose distearate, C₁₈H₃₇CH₂C(O)N(CH₃)-CH₂(CHOH)₄(CH₂OH)₂, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-phospholipid, PEG-cholesterol, PEG-cholesterol derivative, PEG-vitamin A, PEG-vitamin E, random copolymers of vinyl acetate and vinyl pyrrolidone; cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quartemary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10^{™}, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL^{™}, ALKAQUAT^{™}, alkyl pyridinium salts; amines, amine salts, amine oxides, imide azolinium salts, protonated quaternary acrylamides, methylated quaternary polymers, and cationic guar, and/or
(b) the surface stabilizer is selected from the group consisting of hydroxypropylmethylcellulose (HPMC), docusate sodium, and a combination thereof.

13. A composition of any one of claims 1 to 12, additionally comprising at least one nanoparticulate sildenafil free base composition having an effective average particle size of less than about 2 microns, wherein said additional nanoparticulate sildenafil free base composition has an effective average particle size which is different than the particle size of the nanoparticulate sildenafil free base composition of claim 1.

14. A composition of any one of claims 1 to 13, additionally comprising at least one non-sildenafil free base active agent.

15. A composition of claim 14, wherein:
(a) the non-sildenafil free base active agent is selected from the group consisting of alpha adrenergic receptor blocking agents, delaquamine, phenotolamine, doxazosin, prostaglandins, prostoglandin analogs, alprostadil misoprostol, testosterone, antidepressants, trazodone, apomorphine, NO donors, central nervous system stimulants, PDE5 inhibitors, amino acids, proteins, peptides, nucleotides, anti-obesity drugs, nutraceuticals, dietary supplements, central nervous symptom stimulants, carotenoids, corticosteroids, elastase inhibitors, anti-fungals, alkylxanthine, oncology therapies, anti-emetics, analgesics, opioids, antipyretics, cardiovascular agents, anti-inflammatory agents, anthelmintics, anti-arrhythmic agents, antibiotics, anticoagulants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, beta-adrenoceptor blocking agents, blood products, blood substitutes, cardiac inotropic agents, contrast media, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, radio- pharmaceuticals, sex hormones, anti-allergic agents, stimulants, anoretics, sympathomimetics, thyroid agents, vasodilators, vasomodulator, xanthines, Mu receptor antagonists, Kappa receptor antagonists, non-narcotic analgesics, monoamine uptake inhibitors, adenosine regulating agents, cannabinoid derivatives, Substance P antagonists, neurokinin-1 receptor antagonists, and sodium channel blockers; and/or
(b) the non-sildenafil free base active agent is a nutraceutical selected from the group consisting of yohimbine, *Cornus officinalis, Cinnamomum aromaticum, Panax ginseng* and *Pulsatilla pratensis,* and L-arginine; and/or
(c) the non-sildenafil free base inhibitor is selected from the group consisting of vardenafil, tadalafil, TA-1790, UK-114542, Compound 14, EMD221829, EMR 62 203, T-1032, M-54033, M-54018, and E-4010.

16. A composition of any of claims 1 to 15, wherein upon administration the composition redisperses such that the sildenafil free base particles have a particle size selected from the group consisting of less than about 2 microns, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, and less than about 50 nm.

17. A composition of any one of claims 1 to 16, wherein:
(a) upon administration the Tₘₐₓ is less than that of a composition of non-nanoparticulate sildenafil or a composition of nanoparticulate sildenafil citrate, administered at the same dosage; and/or
(b) in comparative pharmacokinetic testing with a composition of non-nanoparticulate sildenafil or a composition of nanoparticulate sildenafil citrate, the nanoparticulate sildenafil free base composition, administered at the same dosage, exhibits a Tₘₐₓ which is selected from the group consisting of less than about 200%, less than about 175%, less than about 150%, less than about 125%, less than about 100%, less than about 90%, less than about 80%, less than about 70%, less than about 60%, less than about 50%, less than about 40%, less than about 30%, less than about 25%, less than about 20%, less than about 15%, and less than about 10% of the Tₘₐₓ exhibited by the composition of non-nanoparticulate sildenafil or the composition of nanoparticulate sildenafil citrate; and/or
(c) the composition has a Tₘₐₓ following administration in a human selected from the group consisting of less than about 1.5 hours, less than about 1.25 hours, less than about 1.0 hours, less than about 50 minutes, less than about 40 minutes, less than about 45 minutes, less than about 35 minutes less than about 30 minutes, less than about 25 minutes, less than about 20 minutes, less than about 15 minutes, and less than about 10 minutes.

18. A composition of any one of claims 1 to 17, wherein:
(a) upon administration the Cₘₐₓ of the composition is greater than the Cₘₐₓ of a composition of non-nanoparticulate sildenafil or a composition of nanoparticulate sildenafil citrate, administered at the same dosage; and/or
(b) in comparative pharmacokinetic testing with a composition of non-nanoparticulate sildenafil or a composition of nanoparticulate sildenafil citrate, the nanoparticulate sildenafil free base composition, administered at the same dosage, exhibits a Cₘₐₓ which is selected from the group consisting of greater than about 5%, greater than about 10%, greater than about 15%, greater than about 20%, greater than about 30%, greater than about 40%, greater than about 50%, greater than about 60%, greater than about 70%, greater than about 80%, greater than about 90%, greater than about 100%, greater than about 110%, greater than about 120%, greater than about 130%, greater than about 140%, and greater than about 150% of the Cₘₐₓ exhibited by the composition of non-nanoparticulate sildenafil or the composition of nanoparticulate sildenafil citrate; and/or
(c) administration of a 100 mg oral dose of the nanoparticulate sildenafil free base composition produces a Cₘₐₓ which is selected from the group consisting of greater than about 440 ng/mL, greater than about 450 ng/mL, greater than about 500 ng/mL, greater than about 550 ng/mL, greater than about 600 ng/mL, greater than about 650 ng/mL, greater than about 700 ng/mL, greater than about 750 ng/mL, greater than about 800 ng/mL, greater than about 850 ng/mL, greater than about 900 ng/mL, greater than about 950 ng/mL, greater than about 1000 ng/mL, greater than about 1050 ng/mL, greater than about 1100 ng/mL, greater than about 1150 ng/mL, greater than about 1200 ng/mL, greater than about 1250 ng/mL, greater than about 1300 ng/mL, about 1350 ng/mL, and greater than about 1400 ng/mL.

19. A composition of any one of claims 1 to 18, wherein administration of a 100 mg oral dose of the nanoparticulate sildenafil free base composition, in a healthy adult male, results in a mean Cₘₐₓ of greater than about 440 ng/mL, a Tₘₐₓ of less than about 60 minutes, or a combination thereof.

20. A composition of any one of claims 1 to 19, wherein:
(a) upon administration the AUC of the composition is greater than the AUC of a composition of non-nanoparticulate sildenafil or a composition of nanoparticulate sildenafil citrate, administered at the same dosage; and/or
(b) in comparative pharmacokinetic testing with a composition of non-nanoparticulate sildenafil or a composition of nanoparticulate sildenafil citrate, the nanoparticulate sildenafil free base composition, administered at the same dosage, exhibits an AUC which is selected from the group consisting of greater than about 5%, greater than about 10%, greater than about 15%, greater than about 20%, greater than about 30%, greater than about 40%, greater than about 50%, greater than about 60%, greater than about 70%, greater than about 80%, greater than about 90%, greater than about 100%, greater than about 110%, greater than about 120%, greater than about 130%, greater than about 140%, and greater than about 150% of the AUC exhibited by the composition of non-nanoparticulate sildenafil or the composition of nanoparticulate sildenafil citrate.

21. A pharmaceutical composition comprising a composition according to any one of claims 1 to 20 in combination with at least one pharmaceutically acceptable excipient.

22. Use of a pharmaceutical composition according to claim 21 for preparation of a medicament.

23. A use of claim 22, wherein the medicament is useful in treating a condition selected from the group consisting of a condition where a selective PDE5 inhibitor is indicated, male erectile dysfunction, impotence, female sexual dysfunction, clitoral dysfunction, female hypoactive sexual desire disorder, female sexual arousal disorder, female sexual pain disorder, female sexual orgasmic dysfunction, sexual dysfunction due to spinal cord injury, premature labor, dysmenorrhea, benign prostatic hyperplasia, bladder outlet obstruction, incontinence, stable angina, unstable angina, variant (Prinzmetal) angina, hypertension, pulmonary hypertension, chronic obstructive pulmonary disease, coronary artery disease, congestive heart failure, atherosclerosis, conditions of reduced blood vessel patency, post-percutaneous transluminal coronary angioplasty, peripheral vascular disease, stroke, nitrate induced tolerance, bronchitis, allergic asthma, chronic asthma, allergic rhinitis, glaucoma, diabetic gastroparesis, pre-eclampsia; Kawasaki's syndrome, nitrate tolerance, multiple sclerosis, diabetic nephropathy, peripheral diabetic neuropathy, Alzheimer's disease, acute respiratory failure, psoriasis, skin necrosis, cancer, metastasis, baldness, nutcracker oesophagus, anal fissure, hemorrhoids, hypoxic vasoconstriction, diseases **characterized by** disorders of gut motility, and irritable bowel syndrome.

24. A method of making a nanoparticulate sildenafil free base composition comprising contacting particles of sildenafil free base with at least one surface stabilizer for a time and under conditions sufficient to provide a composition comprising sildenafil free base particles having an effective average particle size of less than about 2 microns,
wherein the resultant nanoparticulate sildenafil free base composition does not produce significantly different absorption levels when administered under fed as compared to fasting conditions.

25. The method of claim 24, wherein the contacting comprises grinding, wet grinding, homogenization, or precipitation.

## Patentansprüche

1. Zusammensetzung von nanopartikulärer freier Base von Sildenafil, umfassend:
(a) Partikel von freier Base von Sildenafil mit einer effektiven durchschnittlichen Partikelgrösse von weniger als etwa 2000 nm; und
(b) mindestens einen Oberflächenstabilisator,
wobei die Zusammensetzung keine signifikant unterschiedliche Absorptionsspiegel erzeugt, wenn sie nach Nahrungsaufnahme im Vergleich zu nüchternen Bedingungen verabreicht wird.

2. Zusammensetzung nach Anspruch 1, wobei der Unterschied in der:
(a) AUC der erfindungsgemäßen Zusammensetzung von nanopartikulärer freier Base von Sildenafil bei Verabreichung nach Nahrungsaufnahme gegenüber dem nüchternen Zustand ausgewählt ist aus der Gruppe bestehend aus weniger als etwa 35%, weniger als etwa 30%, weniger als etwa 25%, weniger als etwa 20%, weniger als etwa 15%, weniger als etwa 10%, weniger als etwa 5%, weniger als etwa 3% und weniger als etwa 1%; und/oder
(b) Tₘₐₓ der erfindungsgemäßen Zusammensetzung von nanopartikulärer freier Base von Sildenafil bei Verabreichung nach Nahrungsaufnahme gegenüber dem nüchternen Zustand weniger als etwa 60%, weniger als etwa 50%, weniger als etwa 40%, weniger als etwa 30%, weniger als etwa 20%, weniger als etwa 15%, weniger als etwa 10%, weniger als etwa 5% und weniger als etwa 3%; und/oder
(c) Cₘₐₓ der erfindungsgemäßen Zusammensetzung von nanopartikulärer freier Base von Sildenafil bei Verabreichung nach Nahrungsaufnahme gegenüber dem nüchternen Zustand weniger als etwa 50%, weniger als etwa 40%, weniger als etwa 30%, weniger als etwa 20%, weniger als etwa 15%, weniger als etwa 10%, weniger als etwa 5% und weniger als etwa 3% beträgt.

3. Zusammensetzung nach Anspruch 1 oder Anspruch 2, wobei die:
(a) AUC der Zusammensetzung sich nicht signifikant unterscheidet bei Verabreichung nach Nahrungsaufnahme im Vergleich zu nüchternen Bedingungen; und/oder
(b) Tₘₐₓ der Zusammensetzung sich nicht signifikant unterscheidet nach Verabreichung nach Nahrungsaufnahme im Vergleich zu nüchternen Bedingungen; und/oder
(c) Cₘₐₓ der Zusammensetzung sich nicht signifikant unterscheidet nach Verabreichung nach Nahrungsausnahme im Vergleich zu nüchternen Bedingungen.

4. Zusammensetzung nach einem der Ansprüche 1 bis 3, wobei die freie Base von Sildenafil ausgewählt ist aus der Gruppe bestehend aus einer kristallinen Phase, einer amorphen Phase, einer semi-kristallinen Phase, einer semi-amorphen Phase und Mischungen davon.

5. Zusammensetzung nach einem der Ansprüche 1 bis 4, wobei die effektive durchschnittliche Partikelgröße der freien Base von Sildenafil ausgewählt ist aus der Gruppe bestehend aus weniger als etwa 1900 nm, weniger als etwa 1800 nm, weniger als etwa 1700 nm, weniger als etwa 1600 nm, weniger als etwa 1500 nm, weniger als etwa 1400 nm, weniger als etwa 1300 nm, weniger als etwa 1200 nm, weniger als etwa 1100 nm, weniger als etwa 1000 nm, weniger als etwa 900 nm, weniger als etwa 800 nm, weniger als etwa 700 nm, weniger als etwa 600 nm, weniger als etwa 500 nm, weniger als etwa 400 nm, weniger als etwa 300 nm, weniger als etwa 250 nm, weniger als etwa 200 nm, weniger als etwa 100 nm, weniger als etwa 75 nm und weniger als etwa 50 nm.

6. Zusammensetzung nach einem der Ansprüche 1 bis 5, wobei die Zusammensetzung formuliert ist:
(a) zur Verabreichung ausgewählt aus der Gruppe bestehend aus oraler, pulmonaler, rektaler, opthalmischer, kolonaler, parenteraler, intrazisternaler, intravaginaler, intraperitonealer, lokaler, bukkaler, nasaler und topischer Verabreichung; und/oder
(b) in einer Dosierungsform, ausgewählt aus der Gruppe bestehend aus flüssigen Dispersionen, Gelen, Aerosolen, Salben, Cremes, kontrolliert freisetzenden Rezepturen, schnell schmelzenden Rezepturen, lyophilisierten Rezepturen, Tabletten, Kapseln, verzögernd freisetzenden Rezepturen, verlängert freisetzenden Rezepturen, pulsierend freisetzenden Rezepturen, und gemischt unmittelbar freisetzenden und kontrolliert freisetzenden Rezepturen.

7. Zusammensetzung nach einem der Ansprüche 1 bis 6, formuliert in einem Aerosol oder nasalen Spray mit einer Tₘₐₓ, die geringer ist als die, die mit einer Zusammensetzung von nicht-nanopartikulärem Sildenafil beobachtet wird.

8. Zusammensetzung nach einem der Ansprüche 1 bis 7, wobei die Zusammensetzung weiterhin einen oder mehrere weitere pharmazeutisch geeignete Hilfsstoffe, Träger, oder eine Kombination dieser umfasst.

9. Zusammensetzung nach einem der Ansprüche 1 bis 8, wobei:
(a) freie Base von Sildenafil in einer Menge vorliegt, die ausgewählt ist aus der Gruppe bestehend aus von etwa 99,5% bis etwa 0,001 %, von etwa 95% bis etwa 0,1% und von etwa 90% bis etwa 0,5%, bezogen auf das Gewicht, basierend auf dem gesamten kombinierten Trockengewicht der freien Base von Sildenafil und mindestens eines Oberflächenstabilisators, nicht beinhaltend andere Hilfsstoffe; und/oder
(b) mindestens ein Oberflächenstabilisator in einer Menge ausgewählt aus der Gruppe bestehend aus von etwa 0,5% bis etwa 99,999%, von etwa 5,0% bis etwa 99,9%, oder von etwa 10% bis etwa 99,5% bezogen auf das Gewicht, basierend auf dem gesamten kombinierten Trockengewicht der freien Base von Sildenafil und mindestens eines Oberflächenstabilisators, nicht beinhaltend andere Hilfsstoffe, vorliegt.

10. Zusammensetzung nach einem der Ansprüche 1 bis 9, die mindestens zwei Oberflächenstabilisatoren umfasst.

11. Zusammensetzung nach einem der Ansprüche 1 bis 10, wobei der Oberflächenstabilisator ausgewählt ist aus der Gruppe bestehend aus einem nichtionischen Oberflächenstabilisator, einem anionischen Oberflächenstabilisator, einem kationischen Oberflächenstabilisator, einem ionischen Oberflächenstabilisator und einem zwitterionischen Oberflächenstabilisator.

12. Zusammensetzung nach einem der Ansprüche 1 bis 11, wobei:
(a) mindestens ein Oberflächenstabilisator ausgewählt ist aus der Gruppe bestehend aus Cetylpyridiniumchlorid, Gelatine, Casein, Phosphatide, Dextran, Glyzerin, Akaziengummi, Cholesterin, Tragant, Stearinsäure, Benzalkoniumchlorid, Kalziumstearat, Glyzerinmonostearat, Cetostearylalkohol, Cetomakrogol emulgierende Wachse, Sorbitanester, Polyoxyethylenalkylether, Polyoxyethylen- Rizinusölderivate, PolyoxyethylenSorbitanfettsäureester, Polyethylenglykole, Dodecyltrimethylammoniumbromid, Polyoxyethylenstearate, kolloidales Silikondioxid, Phosphate, Natriumdodecylsulfat, Kalzium-Carboxymethylcellulose, Hydroxypropylcellulosen, Hydroxypropylmethylcellulose, Natriumcarboxymethylcellulose, Methylcellulose, Hydroxyethylcellulose, Hydroxypropylmethylcellulosephtalat, nicht-kristalline Cellulose, Magnesiumaluminiumsilikat, Triethanolamin, Polyvinylalkohol, Polyvinylpyrrolidon, 4-(1,1,3,3-Tetramethylbutyl)-Phenolpolymer mit Ethylenoxid und Formaldehyd, Poloxamere, Poloxamine, ein geladenes Phospholipid, Dioctylsulfosuccinat, Dialkylester der Natriumsulfobernsteinsäure, Natriumlaurylsulfat, Alkylarylpolyethersulfonate, Mischungen von Sucrosestearat und Sucrosedistearat, C₁₈H₃₇CH₂C(O)N(CH₃)-CH₂(CHOH)₄(CH₂OH)₂, p-Isononylphenoxypoly-(Glycidol), Decanoyl-N-methylglucamid; n-Decyl β-D-Glucopyranosid, n-Decyl β-D-Maltopyranosid; n-Dodecyl β-D-Glucopyranosid; n-Dodecyl β-D-Maltosid; Heptanoyl-N-methylglucamid; n-Heptyl-β-D-Glucopyranosid; N-Heptyl β-D-thioglucosid; n-hexyl β-D-Glucopyranosid; Nonanoyl-N-methylglucamid; n-Noyl β-D-Glucopyranosid; Octanoyl-N-methylglucamid; n-Octyl- β-D-Glucopyranosid; Octyl β-D-Thioglucopyranosid; Lysozym, PEG-Phospholipid, PEG-Cholesterin, PEG-Cholesterinderivate, PEG-Vitamin A, PEG-Vitamin E, zufällige Copolymere von Vinylacetat und Vinylpyrrolidon; kationische Lipide, Polymethylmethacrylat-trimethylammoniumbromid, Sulfoniumverbindungen, Polyvinylpyrrolidon-2-dimethylaminoethylmethacrylat-dimethylsulfat, Hexadecyltrimethylammoniumbromid, Phosphoniumverbindungen, quartäre Ammoniumverbindungen, Benzyldi-(2-chlorethyl)-ethylammoniumbromid, Kokosnuss Trimethylammoniumchlorid, Kokosnuss Trimethylammoniumbromid, Kokosnuss Methyldihydroxyethylammoniumchlorid, Kokosnuss Methyldihydroxyethylammoniumbromid, Decyltriethylammoniumchlorid, Decyldimethylhydroxyethylammoniumchlorid, Decyldimethylhydroxyethylammoniumchloridbromid, C₁₂-₁₅Dimethylhydroxyethylammoniumchlorid, C₁₂-₁₅Dimethylhydroxyethylammoniumchloridbromid, Kokosnuss Dimethylhydroxyethylammoniumchlorid, Kokosnuss Dimethylhydroxyethylammoniumbromid, Myristyltrimethylammoniummethylsulfat, Lauryldimethylbenzylammoniumchlorid, Lauryldimethylbenzylammoniumbromid, Lauryldimethyl-(ethenoxy)₄-ammoniumchlorid, Lauryldimethyl-(ethenoxy)₄-ammoniumbromid, N-Alkyl(C₁₂₋₁₈)Dimethylbenzylammoniumchlorid, N-Alkyl(C₁₄₋₁₈)Dimethylbenzylammoniumchlorid, N-Tetradecyldimethylbenzylammoniumchlorid Monohydrat, Dimethyldidecylammoniumchlorid, N-Alkyl und (C₁₂₋₁₄)Dimethyl 1-Napthylmethylammoniumchlorid, Trimethylammoniumhalid, Alkyl-trimethylammonium Salze, Dialkyl-dimethylammonium Salze, Lauryltrimethylammoniumchlorid, ethoxyliertes Alkylamidoalkyldialkylammonium Salz, ein ethoxyliertes Trialkylammonium Salz, Dialkylbenzoldialkylammoniumchlorid, N-Didecyldimethylammoniumchlorid, N-Tetradecyldimethylbenzylammoniumchlorid Monohydrat, N-Alkyl(C₁₂-₁₄)Dimethyl-1-naphthylmethylammoniumchlorid, Dodecyldimethylbenzylammoniumchlorid, Dialkylbenzolalkylammoniumchlorid, Lauryltrimethylammoniumchlorid, Alkylbenzylmethylammoniumchlorid, Alkylbenzyldimethylammonimbromid, C₁₂ Trimethylammoniumbromide, C₁₅ Trimethylammoniumbromide, C₁₇ Trimethylammoniumbromide, Dodecylbenzyltriethylammoniumchlorid, Polydiallyldimethylammoniumchlorid (DADMAC), Dimethylammoniumchloride, Alkyldimethylammoniumhalogenide, Tricetylmethylammoniumchlorid, Decyltrimethylammoniumbromid, Dodecyltriethylammoniumbromid, Tetradecyltrimethylammoniumbromid, Methyltrioctylammoniumchlorid, POLYQUAT 10^{™}, Tetrabutylammoniumbromid, Benzyltrimethylammoniumbromid, Cholinester, Benzalkoniumchlorid, Stearalkoniumchloridverbindungen, Cetylpyridiniumbromid, Cetylpyridiniumchlorid, halogenide Salze quartärer Polyoxyethylalkylamine, MIRAPOL^{™}, ALKAQUAT^{™}, Alkylpyridiniumsalze, Amine, Aminsalze, Aminoxide, Imidazoliniumsalze, protonierte quartäre Acrylamide, methylierte quartäre Polymere und kationisches Guar; und/oder
(b) der Oberflächenstabilisator ist ausgewählt aus der Gruppe bestehend aus Hydroxypropylmethylcellulose (HPMC), Natriumdocusat und einer Kombination hiervon.

13. Zusammensetzung nach einem der Ansprüche 1 bis 12, die zusätzlich mindestens eine Zusammensetzung von nanopartikulärer freier Base von Sildenafil, die eine effektive durchschnittliche Partikelgröße von weniger als etwa 2 µm aufweist, umfasst, wobei die zusätzliche Zusammensetzung von nanopartikulärer freier Base von Sildenafil eine effektive durchschnittliche Partikelgröße besitzt, die sich von der Partikelgröße der Zusammensetzung von nanopartikulärer freier Base von Sildenafil nach Anspruch 1 unterscheidet.

14. Zusammensetzung nach einem der Ansprüche 1 bis 13, die zusätzlich mindestens einen aktiven Wirkstoff umfasst, der keine freie Base von Sildenafil ist.

15. Zusammensetzung nach Anspruch 14, wobei:
(a) der aktive Wirkstoff, der keine freie Base von Sildenafil ist, ausgewählt ist aus der Gruppe bestehend aus: Alpha andrenergische Rezeptoren-blockierenden Wirkstoffe, Delaquamin, Phentolamin, Doxazosin, Prostaglandine, Prostaglandinanaloga, Alprostadil Misoprostol, Testosteron, Antidepressiva, Trazodon, Apomorphin, NO Donoren, Stimulantien des Zentralen Nervensystems, PDE5 Inhibitoren, Aminosäuren, Proteine, Peptide, Nucleotide, Anti-Fettsucht Medikamenten, Nährstoffpräparate, diätische Zusatzstoffe, Stimulantien von zentralnervösen Symptomen, Karotinoiden, Corticosteroiden, Elastase-Inhibitoren, Anti-Pilzmittel, Alkylxanthine, Onkologie-Therapeutika, Anti-Brechreiz Mitteln, Analgetica, Opioide, Antifiebermitteln, cardiovasculäre Wirkstoffe, anti-inflammatorische Wirkstoffe, Anthelmintica, Anti-arrhythmische Wirkstoffe, Antibiotika, Anticoagulanzien, antidiabetische Wirkstoffe, Antiepileptika, Antihistamine, antihypertensive Wirkstoffe, antimuskarine Wirkstoffe, antimycobakterielle Wirkstoffe, antineoplastische Wirkstoffe, Immunsuppressiva, antithyroide Wirkstoffe, antivirale Wirkstoffe, Anxiolytika, Sedative, Astringenzien, beta-Adrenorezeptor-blockierende Wirkstoffe, Blutprodukten, Blutsubstitute, Kardiale inotropische Wirkstoffe, Kontrastmittel, Hustenmittel, diagnostische Wirkstoffe, diagnostische bildgebende Wirkstoffe, Diuretika, Dopaminergika, Hämostatika, immunologische Wirkstoffe, lipidregulierende Wirkstoffe, Muskelrelaxantien, Parasympathomimetika, parathyroides Calcitonin und Biphosphonate, Radio-Pharmazeutika, Sexualhormone, anti-allergische Wirkstoffe, Stimulantien, Anoretika, Sympathomimetika, thyroide Wirkstoffe, Vasodilatoren, Vasomodulatoren, Xanthine, Mu-Rezeptor Antagonisten, Kappa-Rezeptor Antagonisten, nicht-narkotisierende Analgetika, Inhibitoren der Monoamin-Aufnahme, Adenosin-regulierende Wirkstoffe, cannabinoide Derivate, P-Substanz-Antagonisten, Neurokinin-1-Rezeptor Antagonisten und Natrium Kanal Blocker; und/oder
(b) der aktive Wirkstoff, der keine freie Base von Sildenafil ist, ist ein "Nutraceutical", ausgewählt aus der Gruppe bestehend aus Yohimbine, *Cornus officinalis, Cinnamomum aromaticum, Panax ginseng* und *Pulsatilla prateinsis* und L-Arginin, und/oder
(c) der Inhibitor, der keine freie Base von Sildenafil ist, ist ausgewählt aus der Gruppe bestehend aus Vardenafil, Tadalafil, TA-1790, UK-114542, Compound 14, EMD221829, EMR 62 203, T-1032,M-54033, M-54018 und E-4010.

16. Zusammensetzung nach einem der Ansprüche 1 bis 15, wobei nach Verabreichung die Zusammensetzung zerfällt, so dass die Partikel aus freier Base von Sildenafil eine Partikelgröße haben die ausgewählt ist aus der Gruppe bestehend aus weniger als etwa 2 µm, weniger als etwa 1900 nm, weniger als etwa 1800 nm, weniger als etwa 1700 nm, weniger als etwa 1600 nm, weniger als etwa 1500, weniger als etwa 1400 nm, weniger als etwa 1300 nm, weniger als etwa 1200 nm, weniger als etwa 1100 nm, weniger als etwa 1000 nm, weniger als etwa 900 nm, weniger als etwa 800 nm, weniger als etwa 700 nm, weniger als etwa 600 nm, weniger als etwa 500 nm, weniger als etwa 400 nm, weniger als etwa 300 nm, weniger als etwa 250 nm, weniger als etwa 200 nm, weniger als etwa 150 nm, weniger als etwa 100 nm, weniger als etwa 75 nm und weniger als etwa 50 nm.

17. Zusammensetzung nach einem der Ansprüche 1 bis 16, wobei;
(a) nach Verabreichung die Tₘₐₓ geringer ist, als die einer Zusammensetzung von nicht-nanopartikulärem Sildenafil oder einer Zusammensetzung von nanopartikulärem Sildenafilcitrat, verabreicht bei gleicher Dosis; und/oder
(b) in vergleichenden pharmakokinetischen Tests mit einer Zusammensetzung von nicht-nanopartikulärem Sildenafil oder einer Zusammensetzung von nanopartikulärem Sildenafilcitrat, die Zusammensetzung von nanopartikulärer freier Base von Sildenafil, verabreicht bei gleicher Dosis, eine Tₘₐₓ aufweist, die ausgewählt ist aus der Gruppe bestehend aus weniger als etwa 200%, weniger als etwa 175%, weniger als etwa 150%, weniger als etwa 125%, weniger als etwa 100%, weniger als etwa 90%, weniger als etwa 80%, weniger als etwa 70%, weniger als etwa 60%, weniger als etwa 50%, weniger als etwa 40%, weniger als etwa 30%, weniger als etwa 25%, weniger als etwa 20%, weniger als etwa 15% und weniger als etwa 10% der Tₘₐₓ, die die Zusammensetzung von nicht-nanopartikulärem Sildenafil oder die Zusammensetzung von nanopartikulärem Sildenafilcitrat aufweist; und/oder
(c) die Zusammensetzung eine Tₘₐₓ nach Verabreichung in einem Menschen hat, welche ausgewählt ist aus der Gruppe bestehend aus weniger als etwa 1,5 Stunden, weniger als etwa 1,25 Stunden, weniger als etwa 1,0 Stunden, weniger als etwa 50 Minuten, weniger als etwa 40 Minuten, weniger als etwa 45 Minuten, weniger als etwa 35 Minuten, weniger als etwa 30 Minuten, weniger als etwa 25 Minuten, weniger als etwa 20 Minuten, weniger als etwa 15 Minuten und weniger als etwa 10 Minuten.

18. Zusammensetzung nach einem der Ansprüche 1 bis 17, wobei:
(a) nach Verabreichung die Cₘₐₓ der Zusammensetzung größer ist als die Cₘₐₓ einer Zusammensetzung von nicht-nanopartikulärem Sildenafil oder einer Zusammensetzung von nanopartikulärem Sildenafilcitrat, verabreicht bei gleicher Dosis; und/oder
(b) in vergleichenden pharmakokinetischen Tests mit einer Zusammensetzung von nicht-nanopartikulärem Sildenafil oder einer Zusammensetzung von nanopartikulärem Sildenafilcitrat, die Zusammensetzung von nanopartikulärer freier Base von Sildenafil, verabreicht bei gleicher Dosis, eine Cₘₐₓ aufweist, die ausgewählt ist aus der Gruppe bestehend aus größer als etwa 5%, größer als etwa 10%, größer als etwa 15%, größer als etwa 20%, größer als etwa 30%, größer als etwa 40%, größer als etwa 50%, größer als etwa 60%, größer als etwa 70%, größer als etwa 80%, größer als etwa 90%, größer als etwa 100%, größer als etwa 110%, größer als etwa 120%, größer als etwa 130%, größer als etwa 140% und größer als etwa 150% der Cₘₐₓ die die Zusammensetzung von nicht-nanopartikulärem Sildenafil oder die Zusammensetzung von nanopartikulärem Sildenafilcitrat aufweist; und/oder
(c) Verabreichen einer oralen Dosis von 100 mg der Zusammensetzung von nanopartikulärer freier Base von Sildenafil eine Cₘₐₓ erzeugt, die ausgewählt ist aus der Gruppe bestehend aus größer als etwa 440 ng/mL, größer als etwa 450 ng/mL, größer als etwa 500 ng/mL, größer als etwa 550 ng/mL, größer als etwa 600 ng/mL, größer als etwa 650 ng/mL, größer als etwa 700 ng/mL, größer als etwa 750 ng/mL, größer als etwa 800 ng/mL, größer als etwa 850 ng/mL, größer als etwa 900 ng/mL, größer als etwa 950 ng/mL, größer als etwa 1000 ng/mL, größer als etwa 1050 ng/mL, größer als etwa 1100 ng/mL, größer als etwa 1150 ng/mL, größer als etwa 1200 ng/mL, größer als etwa 1250 ng/mL, größer als etwa 1300 ng/mL, größer als etwa 1350 ng/mL und größer als etwa 1400 ng/mL.

19. Zusammensetzung nach einem der Ansprüche 1 bis 18, wobei die Verabreichung einer oralen Dosis von 100 mg der Zusammensetzung von nanopartikulärer freier Base von Sildenafil, in einem gesunden erwachsenen Mann, in einer mittleren Cₘₐₓ, die grösser als etwa 440 ng/mL ist, einer Tₘₐₓ von weniger als etwa 60 Minuten, oder einer Kombination hiervon, resultiert.

20. Zusammensetzung nach einem der Ansprüche 1 bis 19, wobei:
(a) nach Verabreichung die AUC der Zusammensetzung größer ist als die AUC einer Zusammensetzung von nicht-nanopartikulärem Sildenafil oder einer Zusammensetzung von nanopartikulärem Sildenafilcitrat, verabreicht bei gleicher Dosis; und/oder
(b) in vergleichenden pharmakokinetischen Tests mit einer Zusammensetzung von nicht-nanopartikulärem Sildenafil oder einer Zusammensetzung von nanopartikulärem Sildenafilcitrat, die Zusammensetzung von nanopartikulärer freier Base von Sildenafil, verabreicht bei gleicher Dosis, eine AUC aufweist, die ausgewählt ist aus der Gruppe bestehend aus größer als etwa 5%, größer als etwa 10%, größer als etwa 15%, größer als etwa 20%, größer als etwa 30%, größer als etwa 40%, größer als etwa 50%, größer als etwa 60%, größer als etwa 70%, größer als etwa 80%, größer als etwa 90%, größer als etwa 100%, größer als etwa 110%, größer als etwa 120%, größer als etwa 130%, größer als etwa 140% und größer als etwa 150% der AUC, die die Zusammensetzung von nicht-nanopartikulärem Sildenafil oder die Zusammensetzung von nanopartikulärem Sildenafilcitrat aufweist.

21. Pharmazeutische Zusammensetzung, die eine Zusammensetzung nach einem der Ansprüche 1 bis 20, in Kombination mit mindestens einem pharmazeutisch geeigneten Hilfsstoff umfasst.

22. Verwendung einer pharmazeutischen Zusammensetzung nach Anspruch 21 zur Herstellung eines Medikamentes.

23. Verwendung nach Anspruch 22, wobei das Medikament in der Behandlung eines Zustandes verwendbar ist, ausgewählt aus der Gruppe bestehend aus einem Zustand, wo ein selektiver PDE5 Inhibitor indiziert ist, männliche Erektionsdysfunktion, Impotenz, weibliche sexuelle Dysfunktion, klitorale Dysfunktion, weibliche hypoaktive Sexualtriebstörung, weibliche sexuelle Erregungsstörung, weibliche sexuelle Schmerzstörung, weibliche sexuelle orgasmische Dysfunktion, sexuelle Dysfunktion zurückzuführen auf Verletzungen des Rückenmarks, verfrühten Wehen, Dysmenorrhoe, gutartige prostatische Hyperplasie, Verlagerung des Blasenausgangs, Inkontinenz, stabile Angina, unstabile Angina, variierende (Prinzmetal-) Angina, Hypertonie, pulmonare Hypertonie, chronische pulmonale Verschlusserkrankungen, Erkrankungen der Koronararterien, kongestive Herzinsuffizienz, Arteriosklerose, Zustände von reduzierter Durchgängigkeit der Blutgefäße, post-perkutane transluminale koronare Angioplastie, periphere Gefäßerkrankung, Schlaganfall, Nitrat-induzierte Toleranz, Bronchitis, allergisches Asthma, chronisches Asthma, allergische Rhinitis, Glaukom, diabetische Gastroparese, Präeklampsie, Kawasaki's Syndrom, Nitrat Toleranz, Multiple Sklerose, diabetische Nephropatie, periphere diabetische Neuropathie, Alzheimer Krankheit, akutes Atemversagen, Psoriasis, Hautnekrose, Krebs, Metastasierung, Haarausfall, Nussknacker-Ösophagus, Analfissur, Hämorrhoiden, hypoxische Vasokonstriktion, Krankheiten charakterisiert durch Störungen der Darmperistaltik, und reizbares Bowel Syndrom.

24. Verfahren zur Herstellung einer Zusammensetzung von nanopartikulärer freier Base von Sildenafil umfassend, das in Kontakt bringen von Partikeln von freier Base von Sildenafil mit mindestens einem Oberflächenstabilisator für eine Zeitdauer und unter Bedingungen, die geeignet sind, eine Zusammensetzung, die Sildenafil freie Base Partikel mit einer effektiven durchschnittlichen Partikelgröße von weniger als etwa 2 µm umfasst, bereit zu stellen, wobei die resultierende Zusammensetzung von nanopartikulärer freier Base von Sildenafil keine signifikant unterschiedliche Absorptionsspiegel bei Verabreichung nach Nahrungsaufnahme im Vergleich zu nüchternen Bedingungen erzeugt.

25. Verfahren nach Anspruch 24, wobei das in Kontakt bringen Mahlen, nasses Mahlen, Homogenisieren oder Präzipitieren umfasst.

## Revendications

1. Composition de base libre de sildénafil nanoparticulaire comprenant:
(a) des particules de base libre de sildénafil ayant une taille moyenne effective de particules de moins d'environ 2000 nm ; et
(b) au moins un stabilisant de surface,
la composition ne produisant pas de niveaux d'absorption significativement différents quand elle est administrée dans des conditions d'alimentation en comparaison de conditions de jeûne.

2. Composition selon la revendication 1, dans laquelle la différence de :
(a) AUC de la composition de base libre de sildénafil nanoparticulaire de l'invention, quand elle est administrée dans l'état alimenté par rapport à l'état de jeûne, est choisie dans le groupe constitué par moins d'environ 35 %, moins d'environ 30 %, moins d'environ 25 %, moins d'environ 20 %, moins d'environ 15 %, moins d'environ 10 %, moins d'environ 5 %, moins d'environ 3 %, et moins d'environ 1 % ; et/ou
(b) Tₘₐₓ pour la composition de base libre de sildénafil nanoparticulaire de l'invention, quand elle est administrée dans l'état alimenté par rapport à l'état de jeûne, est inférieure à environ 60 %, inférieure à environ 50 %, inférieure à environ 40 %, inférieure à environ 30 %, inférieure à environ 20 %, inférieure à environ 15 %, inférieure à environ 10 %, inférieure à environ 5 %, ou inférieure à environ 3 % ; et/ou
(c) Cₘₐₓ pour la composition de base libre de sildénafil nanoparticulaire de l'invention, quand elle est administrée dans l'état alimenté par rapport à l'état de jeûne, est inférieure à environ 50 %, inférieure à environ 40 %, inférieure à environ 30 %, inférieure à environ 20 %, inférieure à environ 15 %, inférieure à environ 10 %, inférieure à environ 5 %, ou inférieure à environ 3 %.

3. Composition selon la revendication 1 ou 2, dans laquelle :
(a) l'AUC de la composition n'est pas significativement différente après administration dans des conditions d'alimentation en comparaison de conditions de jeûne ; et/ou
(b) le Tₘₐₓ de la composition n'est pas significativement différent après administration dans des conditions d'alimentation en comparaison de conditions de jeûne ; et/ou
(c) la Cₘₐₓ de la composition n'est pas significativement différente après administration dans des conditions d'alimentation en comparaison de conditions de jeûne.

4. Composition selon l'une quelconque des revendications 1 à 3, dans laquelle la base libre de sildénafil est choisie dans le groupe constitué par une phase cristalline, une phase amorphe, une phase semi-cristalline, une phase semi-amorphe, et les mélanges de celles-ci.

5. Composition selon l'une quelconque des revendications 1 à 4, dans laquelle la taille moyenne effective de particules de base libre de sildénafil est choisie dans le groupe constitué par moins d'environ 1900 nm, moins d'environ 1800 nm, moins d'environ 1700 nm, moins d'environ 1600 nm, moins d'environ 1500 nm, moins d'environ 1400 nm, moins d'environ 1300 nm, moins d'environ 1200 nm, moins d'environ 1100 nm, moins d'environ 1000 nm, moins d'environ 900 nm, moins d'environ 800 nm, moins d'environ 700 nm, moins d'environ 600 nm, moins d'environ 500 nm, moins d'environ 400 nm, moins d'environ 300 nm, moins d'environ 250 nm, moins d'environ 200 nm, moins d'environ 100 nm, moins d'environ 75 nm, et moins d'environ 50 nm.

6. Composition selon l'une quelconque des revendications 1 à 5, la composition étant formulée :
(a) pour une administration choisie dans le groupe constitué par l'administration orale, pulmonaire, rectale, ophtalmique, colonique, parentérale, intracisternale, intravaginale, intrapéritonéale, locale, buccale, nasale, et topique ; et/ou
(b) sous une forme posologique choisie dans le groupe constitué par les dispersions liquides, les gels, les aérosols, les pommades, les crèmes, les formulations à libération contrôlée, les formulations fondant rapidement, les formulations lyophilisées, les comprimés, les capsules, les formulations à libération retardée, les formulations à libération prolongée, les formulations à libération pulsatile, et les formulations mixtes à libération immédiate et libération contrôlée.

7. Composition selon l'une quelconque des revendications 1 à 6 formulée sous la forme d'un aérosol ou dans un pulvérisateur nasal et ayant un Tₘₐₓ qui est inférieur à celui observé avec une composition de sildénafil non nanoparticulaire.

8. Composition selon l'une quelconque des revendications 1 à 7, la composition comprenant en outre un ou plusieurs excipients ou véhicules pharmaceutiquement acceptables, ou une combinaison de ceux-ci.

9. Composition selon l'une quelconque des revendications 1 à 8, dans laquelle :
(a) la base libre de sildénafil est présente dans une quantité choisie dans le groupe constitué par environ 99,5 % à environ 0,001 %, environ 95 % à environ 0,1 %, et environ 90 % à environ 0,5 %, en poids, par rapport au poids sec total combiné de la base libre de sildénafil et d'au moins un stabilisant de surface, n'incluant pas d'autres excipients ; et/ou
(b) au moins un stabilisant de surface est présent dans une quantité choisie dans le groupe constitué par environ 0,5 % à environ 99,999 %, environ 5,0 % à environ 99,9 %, et environ 10 % à environ 99,5 %, en poids, par rapport au poids sec total combiné de la base libre de sildénafil et d'au moins un stabilisant de surface, n'incluant pas d'autres excipients.

10. Composition selon l'une quelconque des revendications 1 à 9, comprenant au moins deux stabilisants de surface.

11. Composition selon l'une quelconque des revendications 1 à 10, dans laquelle le stabilisant de surface est choisi dans le groupe constitué par un stabilisant de surface non ionique, un stabilisant de surface anionique, un stabilisant de surface cationique, un stabilisant de surface ionique, et un stabilisant de surface zwitterionique.

12. Composition selon l'une quelconque des revendications 1 à 11, dans laquelle :
(a) au moins un stabilisant de surface est choisi dans le groupe constitué par le chlorure de cétylpyridinium, la gélatine, la caséine, les phosphatides, le dextrane, le glycérol, la gomme arabique, le cholestérol, la gomme adragante, l'acide stéarique, le chlorure de benzalkonium, le stéarate de calcium, le monostéarate de glycérol, l'alcool cétostéarylique, la cire émulsifiante au cétomacrogol, les esters de sorbitane, les éthers alkyliques de polyoxyéthylène, les dérivés d'huile de ricin de polyoxyéthylène, les esters d'acides gras de polyoxyéthylène sorbitane, les polyéthylène glycols, le bromure de dodécyltriméthyl-ammonium, les stéarates de polyoxyéthylène, le dioxyde de silicium colloïdal, les phosphates, le dodécylsulfate de sodium, la carboxyméthylcellulose calcique, les hydroxypropylcelluloses, l'hydroxypropyl-méthylcellulose, la carboxyméthylcellulose sodique, la méthylcellulose, l'hydroxyéthylcellulose, le phtalate d'hydroxypropylméthylcellulose, la cellulose non cristalline, l'aluminosilicate de magnésium, la triéthanolamine, l'alcool polyvinylique, la polyvinylpyrrolidone, le polymère de 4-(1,1,3,3-tétraméthylbutyl)phénol avec l'oxyde d'éthylène et le formaldéhyde, les poloxamères ; les poloxamines, un phospholipide chargé, le sulfosuccinate de dioctyle, les esters dialkyliques du sel de sodium d'acide sulfosuccinique, le laurylsulfate de sodium, les alkylarylpolyéthersulfonates, les mélanges de stéarate de saccharose et de distéarate de saccharose, C₁₈H₃₇CH₂C (O)N (CH₃) -CH₂(CHOH)₄(CH₂OH)₂, le p-isononyl-phénoxypoly-(glycidol), le décanoyl-N-méthylglucamide ; le n-décyl-β-D-glucopyranoside ; le n-décyl-β-D-malto-pyranoside ; le n-dodécyl-β-D-glucopyranoside ; le n-dodécyl-β-D-maltoside ; l'heptanoyl-N-méthylglucamide ; le n-heptyl-β-D-glucopyranoside ; le n-heptyl-β-D-thioglucoside ; le n-hexyl-β-D-glucopyranoside ; le nonanoyl-N-méthylglucamide ; le n-noyl-β-D-glucopyranoside ; l'octanoyl-N-méthylglucamide ; le n-octyl-β-D-glucopyranoside ; l'octyl-β-D-thioglucopyranoside ; le lysozyme, le PEG-phospholipide, le PEG-cholestérol, les dérivés de PEG-cholestérol, la PEG-vitamine A, la PEG-vitamine E, les copolymères statistiques d'acétate de vinyle et de vinylpyrrolidone ; les lipides cationiques, le bromure de triméthylammonium de polyméthacrylate de méthyle, les composés de sulfonium, le diméthylsulfate de polyvinylpyrrolidone-2-diméthylaminoéthylméthacrylate, le bromure d'hexadécyl-triméthylammonium, les composés de phosphonium, les composés d'ammonium quaternaire, le bromure de benzyl-di(2-chloroéthyl)éthylammonium, le chlorure de triméthylammonium de noix de coco, le bromure de triméthylammonium de noix de coco, le chlorure de méthyldihydroxyéthylammonium de noix de coco, le bromure de méthyldihydroxyéthylammonium de noix de coco, le chlorure de décyltriéthylammonium, le chlorure de décyldiméthylhydroxyéthylammonium, le chlorobromure de décyldiméthylhydroxyéthylammonium, le chlorure de C₁₂₋₁₅diméthylhydroxyéthylammonium, le chlorobromure de C₁₂₋₁₅diméthylhydroxyéthylammonium, le chlorure de diméthylhydroxyéthylammonium de noix de coco, le bromure de diméthylhydroxyéthylammonium de noix de coco, le méthylsulfate de myristyltriméthylammonium, le chlorure de lauryldiméthylbenzylammonium, le bromure de lauryldiméthylbenzylammonium, le chlorure de lauryl-diméthyl(éthénoxy)₄ ammonium, le bromure de lauryl-diméthyl(éthénoxy)4 ammonium, un chlorure de N-alkyl (C₁₂-₁₈)-diméthylbenzylammonium, un chlorure de N-alkyl (C₁₄-₁₈)-diméthylbenzylammonium, le chlorure de N-tétradécyldiméthylbenzylammonium monohydrate, le chlorure de diméthyldidécylammonium, un chlorure de N-alkyl (C₁₂₋₁₄) - diméthyl-1-naphtylméthylammonium, un halogénure de triméthylammonium, les sels d'alkyl-triméthylammonium, les sels de dialkyl-diméthylammonium, le chlorure de lauryltriméthylammonium, un sel d'alkylamidoalkyldialkylammonium éthoxylé, un sel de trialkylammonium éthoxylé, un chlorure de dialkylbenzènedialkylammonium, le chlorure de N-didécyldiméthylammonium, le chlorure de N-tétradécyldiméthylbenzylammonium monohydrate, un chlorure de N-alkyl(C₁₂₋₁₄)-diméthyl-1-naphtylméthyl-ammonium, le chlorure de dodécyldiméthylbenzylammonium, un chlorure de dialkylbenzènealkylammonium, le chlorure de lauryltriméthylammonium, un chlorure d'alkylbenzyl-méthylammonium, un bromure d'alkylbenzyldiméthyl-ammonium, les bromures de C₁₂-triméthylammonium, les bromures de C₁₅-triméthylammonium, les bromures de C₁₇-triméthylammonium, le chlorure de dodécylbenzyl-triéthylammonium, le poly(chlorure de diallyldiméthyl-ammonium) (DADMAC), les chlorures de diméthylammonium, les halogénures d'alkyldiméthylammonium, le chlorure de tricétylméthylammonium, le bromure de décyltriméthyl-ammonium, le bromure de dodécyltriéthylammonium, le bromure de tétradécyltriméthylammonium, le chlorure de méthyltrioctylammonium, le POLYQUAT 10^{™}, le bromure de tétrabutylammonium, le bromure de benzyltriméthyl-ammonium, les esters de choline, le chlorure de benzalkonium, les composés de chlorure de stéaralkonium, le bromure de cétylpyridinium, le chlorure de cétylpyridinium, les sels d'halogénures de polyoxyéthylalkylamines quaternisées, le MIRAPOL^{™}, l'ALKAQUAT^{™}, les sels d'alkylpyridinium ; les amines, les sels d'amines, les oxydes d'amines, les sels d'imide-azolinium, les acrylamides quaternaires protonés, les polymères quaternaires méthylés, et la gomme guar cationique ; et/ou
(b) le stabilisant de surface est choisi dans le groupe constitué par l'hydroxypropylméthylcellulose (HPMC), le docusate de sodium, et une combinaison de ceux-ci.

13. Composition selon l'une quelconque des revendications 1 à 12, comprenant au moins une composition de base libre de sildénafil nanoparticulaire supplémentaire ayant une taille moyenne effective de particules de moins d'environ 2 microns, dans laquelle ladite composition de base libre de sildénafil nanoparticulaire supplémentaire a une taille moyenne effective de particules qui est différente de la taille de particules de la composition de base libre de sildénafil nanoparticulaire de la revendication 1.

14. Composition selon l'une quelconque des revendications 1 à 13, comprenant en outre au moins un agent actif autre que la base libre de sildénafil.

15. Composition selon la revendication 14, dans laquelle:
(a) l'agent actif autre que la base libre de sildénafil est choisi dans le groupe constitué par les agents bloquant les récepteurs alpha-adrénergiques, la délaquamine, la phénotolamine, la doxazosine, les prostaglandines, les analogues de prostaglandines, l'alprostadil, le misoprostol, la testostérone, les antidépresseurs, la trazodone, l'apomorphine, les donneurs de NO, les stimulants du système nerveux central, les inhibiteurs de la PDE5, les acides aminés, les protéines, les peptides, les nucléotides, les médicaments anti-obésité, les nutraceutiques, les compléments alimentaires, les caroténoïdes, les corticostéroïdes, les inhibiteurs de l'élastase, les antifongiques, les alkylxanthines, les thérapies oncologiques, les antiémétiques, les analgésiques, les opioïdes, les antipyrétiques, les agents cardiovasculaires, les agents anti-inflammatoires, les anthelmintiques, les agents antiarythmiques, les antibiotiques, les anticoagulants, les agents antidiabétiques, les antiépileptiques, les antihistaminiques, les agents antihypertenseurs, les agents antimuscariniques, les agents antimycobactériens, les agents antinéoplasiques, les immunosuppresseurs, les agents antithyroïdiens, les agents antiviraux, les anxiolytiques, les sédatifs, les astringents, les agents bloquant les récepteurs bêta-adrénergiques, les produits sanguins, les substituts du sang, les agents inotropes cardiaques, les milieux de contraste, les antitussifs, les agents de diagnostic, les agents d'imagerie de diagnostic, les diurétiques, les dopaminergiques, les hémostatiques, les agents immunologiques, les agents de régulation des lipides, les relaxants musculaires, les parasympathomimétiques, les biphosphonates et la calcitonine parathyroïdiens, les substances radiopharmaceutiques, les hormones sexuelles, les agents antiallergiques, les stimulants, les anorexigènes, les sympathomimétiques, les agents thyroïdiens, les vasodilatateurs, les vasomodulateurs, les xanthines, les antagonistes du récepteur Mu, les antagonistes du récepteur Kappa, les analgésiques non narcotiques, les inhibiteurs de l'assimilation de monoamines, les agents régulant l'adénosine, les dérivés de cannabinoïdes, les antagonistes de la substance P, les antagonistes du récepteur de neurokinine-1, et les bloqueurs des canaux calciques; et/ou
(b) l'agent actif autre que la base libre de sildénafil est un nutraceutique choisi dans le groupe constitué par la yohimbine, *Cornus officinalis, Cinnamomum aromaticum, Panax ginseng, Pulsatilla pratensis,* et la L-arginine ; et/ou
(c) l'inhibiteur autre que la base libre de sildénafil est choisi dans le groupe constitué par le vardénafil, la tadalafil, le TA-1790, l'UK-114542, le composé 14, l'EMD221829, l'EMR 62 203, le T-1032, le M-54033, le M-54018, et le E-4010.

16. Composition selon l'une quelconque des revendications 1 à 15 dans laquelle, lors d'une administration, la composition se redisperse de telle sorte que les particules de base libre de sildénafil ont une taille de particules choisie dans le groupe constitué par moins d'environ 2 microns, moins d'environ 1900 nm, moins d'environ 1800 nm, moins d'environ 1700 nm, moins d'environ 1600 nm, moins d'environ 1500 nm, moins d'environ 1400 nm, moins d'environ 1300 nm, moins d'environ 1200 nm, moins d'environ 1100 nm, moins d'environ 1000 nm, moins d'environ 900 nm, moins d'environ 800 nm, moins d'environ 700 nm, moins d'environ 600 nm, moins d'environ 500 nm, moins d'environ 400 nm, moins d'environ 300 nm, moins d'environ 250 nm, moins d'environ 200 nm, moins d'environ 150 nm, moins d'environ 100 nm, moins d'environ 75 nm, et moins d'environ 50 nm.

17. Composition selon l'une quelconque des revendications 1 à 16, dans laquelle :
(a) lors d'une administration, le Tₘₐₓ est inférieur à celui d'une composition de sildénafil non nanoparticulaire ou d'une composition de citrate de sildénafil nanoparticulaire, administrée à la même posologie ; et/ou
(b) dans des essais pharmacocinétiques comparatifs avec une composition de sildénafil non nanoparticulaire ou une composition de citrate de sildénafil nanoparticulaire, la composition de base libre de sildénafil nanoparticulaire, administrée à la même posologie, présente un Tₘₐₓ qui est choisi dans le groupe constitué par moins d'environ 200 %, moins d'environ 175 %, moins d'environ 150 %, moins d'environ 125 %, moins d'environ 100 %, moins d'environ 90 %, moins d'environ 80 %, moins d'environ 70 %, moins d'environ 60 %, moins d'environ 50 %, moins d'environ 40 %, moins d'environ 30 %, moins d'environ 25 %, moins d'environ 20 %, moins d'environ 15 %, et moins d'environ 10 %, du Tₘₐₓ affiché par la composition de sildénafil non nanoparticulaire ou la composition de citrate de sildénafil nanoparticulaire ; et/ou
(c) la composition a un Tₘₐₓ après administration à un humain choisi dans le groupe constitué par moins d'environ 1,5 heure, moins d'environ 1,25 heure, moins d'environ 1,0 heure, moins d'environ 50 minutes, moins d'environ 45 minutes, moins d'environ 40 minutes, moins d'environ 35 minutes, moins d'environ 30 minutes, moins d'environ 25 minutes, moins d'environ 20 minutes, moins d'environ 15 minutes, et moins d'environ 10 minutes.

18. Composition selon l'une quelconque des revendications 1 à 17, dans laquelle :
(a) lors d'une administration, la Cₘₐₓ de la composition est supérieure à la Cₘₐₓ d'une composition de sildénafil non nanoparticulaire ou d'une composition de citrate de sildénafil nanoparticulaire, administrée à la même posologie ; et/ou
(b) dans des essais pharmacocinétiques comparatifs avec une composition de sildénafil non nanoparticulaire ou une composition de citrate de sildénafil nanoparticulaire, la composition de base libre de sildénafil nanoparticulaire, administrée à la même posologie, présente une Cₘₐₓ qui est choisie dans le groupe constitué par plus d'environ 5 %, plus d'environ 10 %, plus d'environ 15 %, plus d'environ 20 %, plus d'environ 30 %, plus d'environ 40 %, plus d'environ 50 %, plus d'environ 60 %, plus d'environ 70 %, plus d'environ 80 %, plus d'environ 90 %, plus d'environ 100 %, plus d'environ 110 %, plus d'environ 120 %, plus d'environ 130 %, plus d'environ 140 %, et plus d'environ 150 %, de la Cₘₐₓ affichée par la composition de sildénafil non nanoparticulaire ou la composition de citrate de sildénafil nanoparticulaire ; et/ou
(c) l'administration d'une dose orale de 100 mg de la composition de base libre de sildénafil nanoparticulaire produit une Cₘₐₓ qui est choisie dans le groupe constitué par plus d'environ 440 ng/ml, plus d'environ 450 ng/ml, plus d'environ 500 ng/ml, plus d'environ 550 ng/ml, plus d'environ 600 ng/ml, plus d'environ 650 ng/ml, plus d'environ 700 ng/ml, plus d'environ 750 ng/ml, plus d'environ 800 ng/ml, plus d'environ 850 ng/ml, plus d'environ 900 ng/ml, plus d'environ 950 ng/ml, plus d'environ 1000 ng/ml, plus d'environ 1050 ng/ml, plus d'environ 1100 ng/ml, plus d'environ 1150 ng/ml, plus d'environ 1200 ng/ml, plus d'environ 1250 ng/ml, plus d'environ 1300 ng/ml, plus d'environ 1350 ng/ml, et plus d'environ 1400 ng/ml.

19. Composition selon l'une quelconque des revendications 1 à 18, dans laquelle l'administration d'une dose orale de 100 mg de la composition de base libre de sildénafil nanoparticulaire, à un mâle adulte sain, aboutit à une Cₘₐₓ moyenne de plus d'environ 440 ng/ml, un Tₘₐₓ de moins d'environ 60 minutes, ou une combinaison de ceux-ci.

20. Composition selon l'une quelconque des revendications 1 à 19, dans laquelle :
(a) lors d'une administration, l'AUC de la composition est supérieure à l'AUC d'une composition de sildénafil non nanoparticulaire ou d'une composition de citrate de sildénafil nanoparticulaire ; et/ou
(b) dans des essais pharmacocinétiques comparatifs avec une composition de sildénafil non nanoparticulaire ou une composition de citrate de sildénafil nanoparticulaire, la composition de base libre de sildénafil nanoparticulaire, administrée à la même posologie, présente une AUC qui est choisie dans le groupe constitué par plus d'environ 5 %, plus d'environ 10 %, plus d'environ 15 %, plus d'environ 20 %, plus d'environ 30 %, plus d'environ 40 %, plus d'environ 50 %, plus d'environ 60 %, plus d'environ 70 %, plus d'environ 80 %, plus d'environ 90 %, plus d'environ 100 %, plus d'environ 110 %, plus d'environ 120 %, plus d'environ 130 %, plus d'environ 140 %, et plus d'environ 150 %, de l'AUC affichée par la composition de sildénafil non nanoparticulaire ou la composition de citrate de sildénafil nanoparticulaire.

21. Composition pharmaceutique comprenant une composition selon l'une quelconque des revendications 1 à 20 en combinaison avec au moins un excipient pharmaceutiquement acceptable.

22. Utilisation d'une composition pharmaceutique selon la revendication 21 pour la préparation d'un médicament.

23. Utilisation selon la revendication 22, dans laquelle le médicament est utile dans le traitement d'un état choisi dans le groupe constitué par un état où un inhibiteur sélectif de la PDE5 est indiqué, le dysfonctionnement érectile masculin, l'impuissance, le dysfonctionnement sexuel féminin, le dysfonctionnement clitoridien, le trouble du désir sexuel hypoactif féminin, le trouble de l'excitation sexuelle féminine, le trouble de la douleur sexuelle féminine, le dysfonctionnement orgasmique féminin, le dysfonctionnement sexuel dû à une lésion de la moelle épinière, le travail prématuré, la dysménorrhée, l'hyperplasie bénigne de la prostate, l'obstruction des voies excrétrices, l'incontinence, l'angor stable, l'angor instable, l'angor variant (de Prinzmetal), l'hypertension, l'hypertension pulmonaire, une maladie pulmonaire chronique obstructive, la maladie des artères coronaires, l'insuffisance cardiaque congestive, l'athérosclérose, les états de réduction de la perméabilité des vaisseaux sanguins, les troubles post-angioplastie coronaire transluminale percutanée, la maladie vasculaire périphérique, l'accident vasculaire cérébral, la tolérance induite par les nitrates, la bronchite, l'asthme allergique, l'asthme chronique, la rhinite allergique, le glaucome, la gastroparésie diabétique, la prééclampsie, le syndrome de Kawasaki, la tolérance aux nitrates, la sclérose en plaques, la néphropathie diabétique, la neuropathie diabétique périphérique, la maladie d'Alzheimer, l'insuffisance respiratoire aiguë, le psoriasis, la nécrose cutanée, le cancer, les métastases, la calvitie, l'oesophage casse-noisettes, la fissure anale, les hémorroïdes, la vasoconstriction hypoxique, les maladies **caractérisées par** des troubles de la motilité du tube digestif, et le syndrome de l'intestin irritable.

24. Procédé de fabrication d'une composition de base libre de sildénafil nanoparticulaire comprenant la mise en contact de particules de base libre de sildénafil avec au moins un stabilisant de surface sur une durée et dans des conditions suffisantes pour obtenir une composition comprenant des particules de base libre de sildénafil ayant une taille moyenne effective de particules de moins d'environ 2 microns,
dans lequel la composition de base libre de sildénafil nanoparticulaire résultante ne produit pas de niveaux d'absorption significativement différents quand elle est administrée dans des conditions d'alimentation en comparaison de conditions de jeûne.

25. Procédé selon la revendication 24, dans lequel la mise en contact comprend un broyage, un broyage humide, une homogénéisation, ou une précipitation.
